# EUROPEAN PATENT SPECIFICATION

(11) **EP 3 654 862 B1**
(45) Date of publication and mention of the grant of the patent: **11.02.2026**
(21) Application number: 18755970.3
(22) Date of filing: 17.07.2018
(51) Int. Cl.: A61B 17/80, A61B 17/00, A61B 17/56, A61B 17/72, A61B 34/00, A61B 34/10

(54) **AN IMPLANT FOR TREATING A HUMERUS FRACTURE**
IMPLANTAT FÜR DIE BEHANDLUNG EINER HUMERUS-FRAKTUR
UN IMPLANT POUR LE TRAITEMENT D'UNE FRACTURE DE L'HUMÉRUS

(30) Priority: 17.07.2017 US 201762533203 P
(43) Date of publication of application: 27.05.2020
(73) Proprietor: Mayo Foundation for Medical Education and Research, Rochester, MN 55905 (US)
(72) Inventor: SPERLING, John W., Rochester, MN 55902 (US)
(74) Representative: Samson & Partner Patentanwälte mbB
(86) International application number: PCT/US2018/042489
(87) International publication number: WO 2019/018397

(56) References cited:
- EP-A1- 2 451 372
- WO-A1-97/47251
- US-A1- 2007 123 873
- US-A1- 2009 118 768
- US-A1- 2010 030 277
- US-A1- 2015 190 143
- US-A1- 2017 007 304

## Description

### BACKGROUND OF THE INVENTION

### 1. Field of the Invention

The invention relates to an implant for treating a fracture in a humerus including a greater tuberosity. Also described therein, but not claimed, is a method for modeling the humeral anatomy that facilitates the design of product offerings for shoulder and elbow surgery, manufacturing a periprosthetic implant for repairing a part of a bone in a subject, and a method for the optimization of periprosthetic bone plates and intramedullary nails through the use of medical imaging data from upper extremity periprosthetic fractures.

### 2. Description of the Related Art

There has been a dramatic growth in the number of fractures worldwide with an aging population and proliferation of motor vehicles. There has also been increased patient expectation in regard to function and outcome after sustaining a fracture. Together, these factors have dramatically driven more operative intervention for fractures worldwide. However, current designs of plates and intra-medullary nails are not anatomic in shape and the sizes available are not based on an anatomic distribution.

There is a range of complications in fracture treatment associated with trauma fixation devices that are not anatomically correct. This includes further fracturing the bone when trying to place an intramedullary device that is not in an anatomic shape as well as catastrophic early failure when contact with native bone is not optimized. When looking at plates, current plating systems do not provide for an anatomic offering. For example, all of the proximal and distal humeral plates currently on the market have only one plate width. This results in a plate that is too wide in a significant percentage of the population resulting in soft tissue irritation due to plate overhang. In addition, this results in a plate that is too small in many patients thereby failing to maximize bony fixation and increasing the risk of failure. The rapid growth of international markets with patients representing a spectrum of patient sizes has exacerbated this problem.

Along with the dramatic increase of joint replacements worldwide, there has been an increase incidence of periprosthetic fractures around these implants. Treatment of these fractures is difficult due to the fact that the medullary canal can be filled with an arthroplasty stem making fixation challenging. In addition to fractures around arthroplasty components, similar challenges can occur when fractures occur around intramedullary nails.

In regard to shoulder and elbow arthroplasty, there has also been a rapid increase in the number of product offerings with different humeral stem lengths. In addition to different stem lengths, there are cemented as well as uncemented options for stem fixation. These factors have resulted in different fracture patterns, which were previously not well understood.

In the past, some companies have designed trauma devices by simply overlaying a design over imaging of cadaveric specimens rather than developing a true scientific basis and anatomic rationale for the shape and size distribution of plates and intramedullary devices. In the past, this design process was based on a "best fit" leaving a significant proportion of the population on either side of a bell shape curve with an implant that is either too big or too small.

Current designs of plates and intra-medullary nails are not anatomic in shape and the sizes available are not based on an anatomic distribution. While plates have been specifically designed for use for treatment of lower extremity periprosthetic fractures, there are no fixation systems designed specifically for the upper extremity periprosthetic fractures. Secure fixation with current non-anatomic plates can be very challenging due to inappropriate plate shape, plate length, and screw hole positions that are not optimized to capture the remaining bone. Lower extremity periprosthetic plates tend to be much larger and not-anatomically correct for the upper extremity. Use of these larger lower extremity plates for the upper extremity can necessitate increased soft tissue stripping which can impact healing. Moreover, the use of plates that are not anatomically correct and do not fit correctly can result in impingement on soft tissues and prominence leading to patient discomfort with the need for additional revision surgery.

Therefore, there exists a need for a methodology to improve understanding of periprosthetic fractures and the associated humeral anatomy to facilitate the design and selection of anatomically correct periprosthetic bone plates.
In WO 97/47251 A1, a bone plate adapted for all fracture types of the proximal humerus, especially all three-part and four-part fracture and fracture dislocations of the proximal humerus, is disclosed. The bone plate is configured and arranged to match the contour of a healthy unfractured proximal humerus, and includes an elongate shaft portion adapted for receiving bone screws to fix the bone plate to a shaft of a humerus, and a head portion including a first head section and a second head section with an obtuse angle defined therebetween.
In US 2017/056081 A1, a system for proximal humeral stabilization including a bone plate having an elongated portion extending along a longitudinal axis and an enlarged head portion extending from the elongated portion is disclosed.

### SUMMARY OF THE INVENTION

The present invention is defined in claim 1 while preferred embodiments are set forth in the dependent claims.

Associated surgical methods are also described herein to aid understanding the invention. These methods do not form part of the claimed invention.

Methods for manufacturing an orthopedic implant are also described herein. These methods do not form part of the claimed invention.

The humerus is not straight and the use of non-anatomically correct devices can result in catastrophic early device loosening when contact with native bone is not optimized, iatrogenic humeral fractures, and increased risk of future fractures due to stress risers. The present invention addresses the foregoing needs. Disclosed herein are methods to improve understanding of the associated humeral anatomy. This methodology describes the interaction of anatomical features of the humerus and how these features change based on the specific location in the humerus. Additionally, the methodology has demonstrated that the shape of the humerus is side specific. Therefore, having right and left specific devices with an anatomic shape in a true population based distribution may further facilitate and improve device design. The methodology can optimize loading and fit at the bone-device interface. This methodology can be used to facilitate the design of total and partial elbow arthroplasty, intramedullary nails for the humerus, revision length stems for shoulder arthroplasty, plates for periprosthetic humeral fractures, mid-shaft humeral fractures and distal humeral fractures. The methodology and associated data set can further help define the appropriate size, shape, distribution of product offerings for these devices.

In addition to understanding the associated humeral anatomy, studying and analysis of periprosthetic fractures may facilitate the design and selection of anatomically correct periprosthetic bone plates.

In one aspect, the disclosure provides a method where improved understanding of humeral anatomy is gained through the use of CT scan data and 3D modeling. Whereas previous reports noted the potential bending of the humerus one plane, the current methodology notes that the humeral anatomy has a specific three dimensional architecture. The methodology, therefore, can facilitate the design of anatomically correct implants that minimize potential complications.

In one configuration, a device is provided for treating a fracture in a bone of a subject. The device includes a first section having a first longitudinal axis and a second section having a second longitudinal axis. The first section is connected to the second section at a first junction between the first section and the second section. The first longitudinal axis and the second longitudinal axis form an oblique angle at the junction and the second section has a terminal end section having a width greater than the first section. The first section and the second section of the device may be configured to provide fixation to a region of the bone and includes a plurality of screw holes.

In some configurations of the device, the bone is the humerus and the second end section is adapted to conform to an outer surface of a proximal end section of the bone. A perimeter of the terminal end of the second section may be dimensioned to conform to a greater tuberosity of the proximal end section of the humerus. The oblique angle formed at the junction may be configured to match an angle of a greater tuberosity from a centerline of the humerus. A length of the device may also be determined by a length of the humerus.

In some configurations of the device, a width of the first section is configured to provide fixation for a fracture in the bone. A plurality of screw holes may be provided on the device and the number of screw holes may be correlated to the width of the terminal end of the second section. The device may also be configured to be specific for a left and a right side of the subject.

In some configuration, the device further includes a third section having a third longitudinal axis. The first section is connected to the third section at a second junction line between the first section and the third section. The first longitudinal axis and the third longitudinal axis may form an oblique angle at the second junction.

In some configurations, the bone is the humerus and the second end section is adapted to conform to an outer surface of a distal end section of the bone. A perimeter of the terminal end of the second section may be dimensioned to conform to a condyle of the distal end section of the humerus. The oblique angle formed at the first junction may be configured to match an angle of a condyle from a centerline of the humerus. The oblique angle formed at the second junction may be configured to match an angle of a condyle from a centerline of the humerus. A length of the device may be determined by a length of the humerus. A length of the third section may be configured to provide fixation for a fracture in the bone.

In one configuration, a device for treating a fracture in a bone is provided. The device includes a first section having a first longitudinal axis and a second section having a second longitudinal axis. The first section is connected to the second section at a first junction between the first section and the second section. The first junction forms a transition portion dimensioned to provide a curvature connecting the first section to the second section.

In some configurations, the location of the first junction is determined by a location of greatest deviation from a straight centerline of the bone. The first section has a length that may be greater than a length of the second section.

In some configurations, the device may include an intramedullary (IM) nail and the bone may be a humerus. The location of the first junction may be between 60-90 percent of the length of the humerus. The location of the first junction may also be at 80 percent of the length of the humerus.

In some configurations, the device includes a third section having a third longitudinal axis. The second section is connected to the third section at a second junction between the second section and the third section. The second junction forms a transition portion dimensioned to provide a curvature connecting the second section to the third section. The location of the second junction may be determined by a location of deviation from a straight centerline of the bone. The second section has a length that may be greater than a length of the third section and the first section. The device may be an intramedullary (IM) nail and the bone is a humerus. When the bone is the humerus, the location of the second junction may be between 10-30 percent of the length of the humerus or may be at 20 percent of the length of the humerus.

In one configuration, a method is provided for manufacturing an orthopedic implant for repairing a part of a bone in a subject. The method includes forming the implant to include at least one bend wherein the at least one bend corresponds to an anatomic shape. The anatomic shape may be determined by a number of steps, which may include: i) obtaining an image of the bone from at least one viewing plane; ii) orienting on the image a first reference line indicating a maximum width of a feature of the bone from a first border of the bone to an opposite second border of the bone; iii) orienting on the image a second reference line perpendicular to the first reference line and extending from a midpoint of the first reference line to an edge of the bone indicating a length of the feature of the bone; iv) orienting on the image a third reference line indicating a length from a centerline of the bone to the midpoint of the first reference line; v) determining an angle between the third reference line and the second reference line to determine the at least one bend of the implant.

In some configurations, the method includes where the implant is at least one of a periprosthetic bone plate, a proximal humeral plate, a distal humeral plate, a humeral nail, or a humeral stem. The centerline used in the method may be: 1) a line with a constant equal distance between the first border and the second border of the bone, which are cortical bone borders; or 2) a line with a constant equal distance between the first border and the second border of the bone, which are cancellous bone borders, or 3) a straight longitudinal bone axis centerline.

In some configurations, the bone used in the method is the humerus. When the bone is the humerus, the feature may be a greater tuberosity, and the first reference line indicates a width of the greater tuberosity on the humerus. One bend of the implant may correspond to an angle of a greater tuberosity from the centerline of the humerus. In some configurations when the bone is the humerus, the feature is a condyle, and the first reference line indicates a width of the condyle on the humerus. One bend of the implant may correspond to an angle of the condyle from the centerline of the humerus.

In some configurations, the bone used in the method may be the femur, tibia, radius, or the ulna. The image used in the method may be a computed tomography scan slice. In some configurations, the method includes determining a thickness of the bone to determine a screw hole location on the implant. The method may be automated, such that images are sent to a control system having a processor configured to execute a program stored thereon to automatically extract measurements of the bone of the subject. The automated measurements of the bone may be referenced to manufacture a plate using an additive manufacturing system.

In one configuration, a method is provided for manufacturing an orthopedic implant for repairing a part of a bone in a subject. The method may include forming the implant to include at least two bends. The bends may correspond to an anatomic shape determined by: i) obtaining an image of the bone from at least one viewing plane; ii) orienting on the image a first reference line indicating a maximum width of a first feature of the bone from a first border of the bone to an opposite second border of the bone; iii) orienting on the image a second reference line indicating a maximum width of a second feature of the bone from a first border of the bone to an opposite second border of the bone; iv) orienting on the image a third reference line perpendicular to the first reference line and extending from a midpoint of the first reference line to a midpoint of the second reference line; v) orienting on the image a fourth reference line perpendicular to the second reference line and extending from a midpoint of the second reference line to an edge of the bone indicating a length of the second feature of the bone; vi) orienting on the image a fifth reference line indicating a length from a centerline of the bone to the midpoint of the first reference line; vii) determining an angle between the third reference line and the fourth reference line to determine at least one bend of the implant; and viii) determining an angle between the third reference line and the fifth reference line to determine at least one bend of the implant.

In some configurations, the method includes when the feature is a condyle of a humerus, and the first reference line indicates a width of the condyle on the humerus at an olecranon fossa. The second reference line may then indicate a maximum width of the condyle.

In one configuration, a method is provided for manufacturing an orthopedic implant for repairing a part of a bone in a subject. The method includes forming the implant to include at least one bend where the bend corresponds to an anatomic shape determined by: i) obtaining an image of the bone from at least one viewing plane; ii) orienting on the image a first reference line indicating a maximum width of a feature of the bone from a first border of the bone to an opposite second border of the bone; iii) orienting on the image a second reference line perpendicular to the first reference line and extending from a midpoint of the first reference line to a centerline of the bone indicating a length of the feature of the bone; and iv) determining an angle between the second reference line and the centerline to determine the at least one bend of the implant. In some configurations, the bone used in the method is the humerus. When the bone is the humerus, the feature may be a greater tuberosity, and the first reference line indicates a width of the greater tuberosity on the humerus. One bend of the implant may correspond to an angle of a greater tuberosity from the centerline of the humerus.

In one aspect, the disclosure provides a method for manufacturing a periprosthetic implant for repairing a part of a bone in a subject. The method can comprise forming the periprosthetic implant to include at least one curved surface, a contour of the at least one curved surface corresponds to an anatomic shape. The anatomic shape can be determined by a number of steps which may include (i) obtaining an image of the bone from at least one viewing plane, where viewing planes may include sagittal, coronal, and axial viewing planes. (ii) orienting on the image a proximal aspect line that extends from a first border of the bone to an opposite second border of the bone, (iii) orienting on the image a longitudinal bone axis extending from the proximal aspect line along a length of the bone between the first border and second border, (iv) orienting on the image a plurality of lateral lines at different distances from the proximal aspect line, each of the plurality of lateral lines extending perpendicularly from one of a plurality of first intersection points on the first border of the bone to one of a plurality of second intersection points intersecting the longitudinal bone axis at one of a plurality of second intersection points, and (v) extrapolating the anatomic shape based on the plurality of first intersection points and the plurality of second intersection points.

In some aspects, the periprosthetic implant can be a periprosthetic bone plate. The plurality of lateral lines can be placed at equidistant intervals distally from the proximal aspect line, or from a bone cut line. The equidistant interval can be in a range of 0.1 to 50 millimeters.

In some aspects, the bone can be the humerus. In other aspects, the bone can be the radius, the ulna, or any other bone.

In some aspects, the image can be a computed tomography scan slice. Step (v) can further comprise measuring a first reference distance of a first line of the at least three lateral lines, the first line extending perpendicularly from a first point of the plurality of first intersection points to a first point of the plurality of second intersection points; measuring a second reference distance of a second line of the at least three lateral lines, the second line extending perpendicularly from a second point of the plurality of first intersection points to a second point of the plurality of second intersection points; measuring a third reference distance of a third line of the at least three lateral lines, the third line extending perpendicularly from a third point of the plurality of first intersection points to a third point of the plurality of second intersection points; and extrapolating the anatomic shape of the first border based on the first reference distance, the second reference distance, and the third reference distance.

In some aspects, step (v) can further comprise: extrapolating a first curvature of the anatomic shape between the first point of the plurality of first intersection points and the second point of the plurality of first intersection points based on the first reference distance and the second reference distance; and extrapolating a second curvature of the anatomic shape between the second point of the plurality of first intersection points and the third point of the plurality of first intersection points based on the second reference distance and the third reference distance.

In some aspects, step (v) can further comprise extrapolating a curvature of the anatomic shape with data from the sagittal viewing plane, or extrapolating a curvature of the anatomic shape with data from the coronal viewing plane, or extrapolating a curvature of the anatomic shape with data from the axial viewing plane. Any combination of one or more viewing planes may be used to determine the curvature of the anatomy, and the viewing planes used may be obtained at non-orthogonal angles to each other.

In some aspects, the bone can include a periprosthetic fracture. The periprosthetic implant can be formed to fit the anatomic shape of the bone and to correct the periprosthetic fracture. The periprosthetic fracture can be characterized by (i) determining a length and width of a prosthetic stem, (ii) determining a geometry of the stem, (iii) determining a fixation of the stem, (iv) determining the fracture pattern, (v) determining if the fracture pattern is comminuted, (vi) determining the amount of angulation and displacement, and (vii) classifying the displacement. The periprosthetic implant can be formed having a length, width, and a shape, the length, width, and shape being determined by the characterized periprosthetic fracture.

In another aspect, the disclosure provides a device for treating a fracture between a proximal section of a bone and a distal section of the bone wherein the proximal section of the bone has a prosthesis implanted therein for shoulder arthroplasty, or where the distal section of the bone has a prosthesis implanted therein for elbow arthroplasty. The device comprises an elongated plate dimensioned for placement on the bone across the fracture. The plate has a bone interface surface that faces the bone when the plate is placed on the bone across the fracture. In some embodiments, the bone interface surface has a shape that transitions from a first curvature that is convex or concave at a proximal portion of the plate to a second curvature at a second portion of the plate longitudinally adjacent to the proximal portion of the plate. The second curvature is convex when the first curvature is concave, and the second curvature is concave when the first curvature is convex. The shape of the bone interface surface may transition from the second curvature to a third curvature at a distal portion of the plate longitudinally adjacent to the second portion of the plate. The third curvature is convex when the second curvature is concave, and the third curvature is concave when the second curvature is convex. The first curvature may be convex such that the second curvature is concave and the third curvature is convex. One skilled in the art will appreciate that the shape of a plate may be defined by any number of curvature sections.

In some aspects, the plate can be a proximal humeral short periprosthetic plate formed for a short stem with a minimal distal fracture extension.

In some aspects, the plate can be a proximal humeral long periprosthetic plate formed for at least one of short stems with a distal fracture extension and a regular length stem with a minimal distal extension.

In some aspects, the plate can be a short distal humeral periprosthetic plate formed for a short humeral stem used with a total elbow arthroplasty, with a minimal proximal fracture extension.

In some aspects, the plate can be a long distal humeral periprosthetic plate, formed for a short humeral stem used with at least one of a total elbow arthroplasty with a proximal humeral fracture extension and a regular length humeral stem used with a total elbow arthroplasty with a minimal proximal fracture extension.

In some aspects, the plate can be a midshaft humeral periprosthetic plate formed for at least one of a regular length shoulder/humeral stems with a minimal fracture extension and a midshaft nonperiprosthetic fracture.

In some aspects, the plate can be a full-length periprosthetic plate formed for fractures that encompass a significant portion of the humerus, including highly comminuted fractures.

Features and advantages of the present invention will become better understood upon consideration of the following detailed description, drawings, and appended claims.

### BRIEF DESCRIPTION OF THE DRAWINGS

FIG. 1 is a cross-sectional view of one embodiment of a prior art shoulder prosthesis suitable for use in the present disclosure.
FIG. 2 shows a traced computed tomography (CT) two-dimensional (2D) CT slice in a coronal viewing plane of the humerus with measurement lines shown in dashed lines.
FIG. 2A shows a traced computed tomography (CT) two-dimensional (2D) CT slice in a coronal viewing plane of the humerus.
FIG. 2B shows a traced computed tomography (CT) two-dimensional (2D) CT slice in a sagittal viewing plane of the humerus with measurement lines shown in dashed lines.
FIG. 2C shows a traced computed tomography (CT) two-dimensional (2D) CT slice in a sagittal viewing plane of the humerus.
FIG. 2D shows a coronal viewing plane of a humerus with measurement lines shown.
FIG. 2E shows an axial cross section of a humerus and a depiction of the cross section's location on an image of a humerus bone with measurement lines shown.
FIG. 3A shows a sagittal view of a proximal section of a humerus with measurement lines shown.
FIG. 3B shows a sagittal view of a proximal section of a humerus with measurement lines shown.
FIG. 3C shows a proximal section of a humerus with measurement lines shown.
FIG. 3D shows a proximal section of a humerus with radius of curvature lines shown.
FIG. 4A shows a traced computed tomography (CT) two-dimensional (2D) CT slice in an axial viewing plane of the humerus along the line 4A-4A in FIG. 2 with a periprosthetic bone plate installed.
FIG. 4B shows a traced computed tomography (CT) two-dimensional (2D) CT slice in an axial viewing plane of the humerus along the line 4B-4B in FIG. 2 with a periprosthetic bone plate installed.
FIG. 4C shows a traced computed tomography (CT) two-dimensional (2D) CT slice in an axial viewing plane of the humerus along the line 4C-4C in FIG. 2 with a periprosthetic bone plate installed.
FIG. 4D shows a traced computed tomography (CT) two-dimensional (2D) CT slice in an axial viewing plane of the humerus along the line 4D-4D in FIG. 2 with a periprosthetic bone plate installed.
FIG. 4E shows a traced computed tomography (CT) two-dimensional (2D) CT slice in an axial viewing plane of the humerus along the line 4E-4E in FIG. 2 with a periprosthetic bone plate installed.
FIG. 4F shows a traced computed tomography (CT) two-dimensional (2D) CT slice in an axial viewing plane of the humerus along the line 4F-4F in FIG. 2 with a periprosthetic bone plate installed.
FIGs. 5A, 5B and 5C show one embodiment of a bone plate used with a shoulder prosthesis.
FIG. 6 shows one embodiment of a bone plate used with a shoulder prosthesis in a coronal (AP) viewing plane.
FIG. 7 shows one embodiment of a bone plate used with a shoulder prosthesis in a sagittal viewing plane.
FIG. 8 shows a perspective view of one embodiment of a periprosthetic bone plate.
FIG. 9A, 9B, 9C and 9D show a lateral condyle with measurement lines shown.
FIG. 10A, 10B, and 10C show a posterior lateral condyle with measurement lines shown.
FIGs. 11A, 11B, 11C and 11D show a medial condyle with measurement lines shown.
FIGs. 12A, 12B and 12C show a posterior medial condyle with measurement lines shown.
FIGs. 13A, 13B, and 13C show embodiments of a lateral condyle plate.
FIGs. 14A, 14B, and 14C show embodiments of a posterolateral condyle plate.
FIGs. 15A, 15B, and 15C show embodiments of a medial condyle plate.
FIGs. 16A, 16B, and 16C show embodiments of a posteromedial condyle plate.
FIG 17 shows a graphical representation of averaged cancellous centerline offsets.
FIGs. 18A and 18B show embodiments of a humeral nail.
FIGs. 19A, 19B 19C and 19D show graphical representations of width measurements acquired for humeral bones.

Like reference numerals will be used to refer to like parts from Figure to Figure in the following description of the drawings.

### DETAILED DESCRIPTION OF THE INVENTION

In one aspect of the present disclosure, a novel methodology to improve understanding of external and internal anatomy of bones through the use of CT scan data and 3D modeling is provided. A retrospective review was undertaken of the largest consecutive series of shoulder and elbow periprosthetic fractures to improve understanding of periprosthetic fractures. This review further reinforced the need as well as benefit of a truly anatomic plating system that can be adaptable to address periprosthetic fractures. While the methodology is described in detail for the humerus, this methodology is applicable to other bones including, but not limited to the femur, tibia, radius, ulna, vertebral bodies, etc. This methodology describes the interaction of anatomical features of the external and internal humerus and how these features change based on the specific location in the humerus. In one aspect of the present disclosure, in order to drive efficiency automated measurements were performed and this automation may be applicable to any bone.

In one aspect of the present disclosure, plate and intramedullary nail models were created to test the methodology and the interaction of the anatomic features and their interdependence on each other. The models had specific features for improved anatomic fracture fixation for intramedullary nails as well as plates. The results of the testing validated that the methodology significantly improved fit compared to currently available designs. The methodology can optimize and facilitate the design of truly anatomic trauma fixation devices in an anatomic shape as well as size distribution.

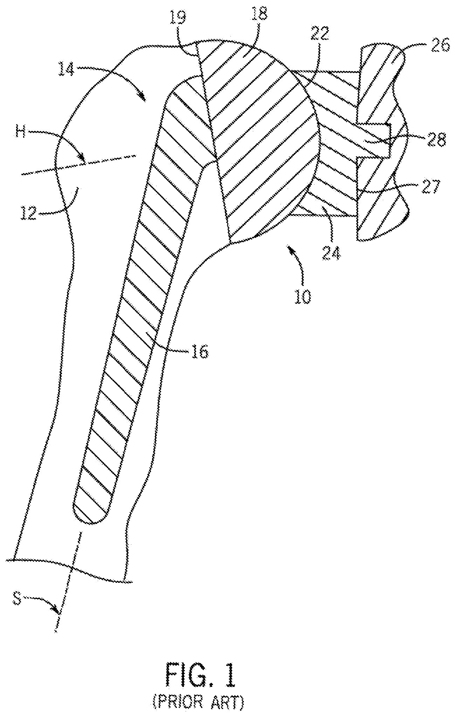

Looking first at FIG. 1, there is shown one example embodiment of a prior art anatomic total shoulder prosthesis 10. One skilled in the art will appreciate that other prostheses, such as reverse shoulder arthroplasty, hemi arthroplasty, stemless shoulder arthroplasty, resurfacing, elbow prostheses and the like may be suitable for use with the present disclosure. The upper portion of the humerus 12 is replaced by a humeral component 14 including a stem 16 that extends into a bore formed within the humerus 12. Typically, the stem 16 is fixed within the bore formed within the humerus 12. The stem 16 has a longitudinal stem axis S. A generally hemispherical head 18 is connected to the stem 16. The stem 16 can be monolithic with the head 18, or the stem 16 and the head 18 can formed as separate parts. The hemispherical head 18 has a base surface 19 and a longitudinal head axis H. The hemispherical head 18 of the humeral component 14 articulates with a complementary concave section 22 of a glenoid component 24 that is fixed within the glenoid cavity of the scapula 26 (shown cutaway) using cemented or uncemented posts 28. The glenoid component 24 includes a base surface 27 opposite the concave section 22 that serves as an articular surface of the glenoid component 24.

A unique database of fifty consecutive high resolution thin cut two dimensional and three dimensional CT scans of the entire humerus with a custom designed bone stock protocol of patients who have undergone anatomic shoulder arthroplasty was available for study. This custom designed protocol was specifically developed at the Mayo Clinic for a detailed understanding of the anatomy of these patients. In addition, 3D modeling of each of these patients was performed. A method for understanding external and internal humeral anatomy was subsequently developed using this unique resource and underwent validation. This data set facilitated developing a methodology to understand the humeral anatomy and facilitate the design of anatomically correct plates and implants.

In non-limiting examples, the method facilitates the design of total and partial elbow arthroplasty, intramedullary nails for the humerus, revision length stems for shoulder arthroplasty, plates for periprosthetic humeral fractures, mid-shaft humeral fractures and distal humeral fractures.

Proper design and/or selection of a periprosthetic plate can be achieved using a method of this disclosure. The proper design and selection of a periprosthetic plate was facilitated by review of a large volume of upper extremity prosthetic replacements, specifically shoulder and elbow replacements. Patients with periprosthetic humeral fractures around a shoulder arthroplasty or elbow arthroplasty were identified. This unique data set included 108 cases: 59 shoulder arthroplasties with periprosthetic humeral fractures and 49 elbow arthroplasties with periprosthetic humeral fractures. This data set facilitated understanding the specific fracture location, fracture pattern, and remaining bone stock. Accordingly, in one aspect, a method of designing and manufacturing a periprosthetic implant for repairing a part of a bone in a subject was developed. Specifically, a method for designing and manufacturing a periprosthetic implant for periprosthetic fractures around shoulder arthroplasty and periprosthetic fractures around elbow arthroplasty was developed.

### Periprosthetic Fractures around Shoulder Arthroplasty

A prosthetic implant present in a subject can be characterized by a number of parameters. A length and width of a stem of the prosthetic implant can be measured in millimeters. A geometry of the stem could be assessed as either tapered or cylindrical. A fixation mechanism of the stem could be determined to be uncemented or cemented. An upper extremity fracture in a subject can have a variety of patterns that can be assessed as one of the following: (1) greater tuberosity only; (2) type-A fractures are located at the tip of the prosthesis and extend proximally; (3) type-B fractures lie at the tip of the prosthesis without extension or with only minimal extension proximally but can have a variable amount of extension distally; and (4) type-C fractures are located distal to the tip of the prosthesis. The fracture can also be determined as a comminuted or not comminuted fracture. It is also important to determine if the stem has become loose. The fracture pattern can be further characterized as transverse, oblique, or spiral, and amounts of angulation and displacement can also be assessed. Angulation was classified as mild (≤15°), moderate (15° to 30°), or severe (>30°). Displacement was classified as mild (within one-third of the diameter of the humeral shaft), moderate (one-third to two-thirds of the diameter of the humeral shaft), or severe (beyond two-thirds of the diameter of the humeral shaft), or complete displacement. The greater tuberosity was assessed to determine if an adequate amount of bone was present in the greater tuberosity for screw fixation. Treatment was determined as surgical or non-surgical and treatment type was also determined. Table A displays the data for 59 shoulder arthroplasties with periprosthetic humeral fractures classified according to the above criteria.

**Table A**

| Humeral Stem Width | Stem Geometry | Stem Fixation | Fracture Type | Comminuted |
|---|---|---|---|---|
| 4 mm-8 mm (25) | Taper (36) | Uncemented (35) | Greater Tuberosity (6) | Yes (31) |
| 9 mm-13 mm (22) | Cylindrical (23) | Cemented (24) | Type A (17) | No (28) |
| 14 mm-18 mm (12) | | | Type B (22) | |
| | | | Type C (13) | |
| | | | | |

| Humeral Stem Loose | Fracture Pattern | Fracture Angulation | Displacement | Bone Present for Fixation |
|---|---|---|---|---|
| Yes (9) | Transverse (27) | <15° (28) | Within 1/3 diameter humeral shaft (24) | Yes (43) |
| No (50) | Oblique (23) | 15-30° (14) | 1/3 to 2/3 diameter humeral shaft (9) | No (16) |
| | Spiral (9) | >30° (17) | Greater 2/3 diameter humeral shaft (26) | |

### Periprosthetic Fractures around Elbow Arthroplasty

A prosthetic implant present in a subject can be characterized by a number of parameters. A length and width of a stem of the prosthetic implant can be measured in millimeters. A geometry of the stem could be assessed as either tapered or cylindrical. A fixation mechanism of the stem could be determined to be uncemented or cemented. An upper extremity fracture in a subject can have a variety of patterns that can be assessed as one of the following: (1) condylar region only; (2) type-A fractures are located at the tip of the prosthesis and extend toward distal humerus, (3) type-B fractures lie at the tip of the prosthesis without extension or with only minimal extension distally but can have a variable amount of extension proximally, and (4) type-C fractures are located proximal to the tip of the prosthesis. The fracture can also be determined as a comminuted or not comminuted fracture. It is also important to determine if the stem has become loose. The fracture pattern can be further characterized as transverse, oblique, or spiral, and amounts of angulation and displacement can also be assessed. Angulation was classified as mild (≤15°), moderate (15° to 30°), or severe (>30°). Displacement was classified as mild (within one-third of the diameter of the humeral shaft), moderate (one-third to two-thirds of the diameter of the humeral shaft), or severe (beyond two-thirds of the diameter of the humeral shaft), or complete displacement. Treatment was determined as surgical or non-surgical and treatment type was also determined. Table B displays the data for 49 elbow arthroplasties with periprosthetic humeral fractures classified according to the above criteria.

**Table B**

| Humeral Stem Length | Stem Width | Cement Fixation | Fracture Type | Comminuted |
|---|---|---|---|---|
| 70 mm-100 mm (31) | 4 mm (6) | Cemented (48) | Condylar (1) | No (26) |
| 120 mm-150 mm (18) | 6 mm (37) | Uncemented (1) | Type A (1) | Yes (23) |
| | 8 mm (6) | | Type B (24) | |
| | | | Type C (23) | |
| | | | | |

| Stem Loose | Fracture Pattern | Angulation | Displacement | |
|---|---|---|---|---|
| No (23) | Transverse (14) | <15° (7) | Within 1/3 diameter humeral shaft (7) | |
| Yes (26) | Oblique (25) | 15-30° (15) | 1/3 to 2/3 diameter humeral shaft (13) | |
| | Spiral (10) | >30° (27) | Greater 2/3 diameter humeral shaft (29) | |

### Pre-Operative CT Scans

The methodology for facilitating the design and manufacturing of anatomically correct plates and implants involves analysis of pre-operation medical imaging data, such as from computerized tomography (CT) scans, and may also include the use of 3D models. In one aspect, a periprosthetic implant can be formed to include at least one curved surface that can have contours that correspond to anatomic shapes of the subject. A contour of the at least one curved surface can correspond to an anatomic shape having been determined during analysis of CT scans of the subject.

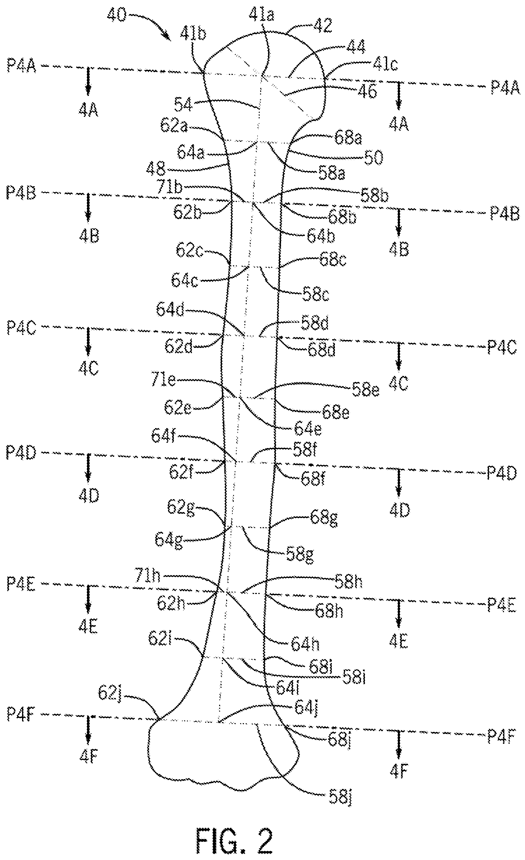

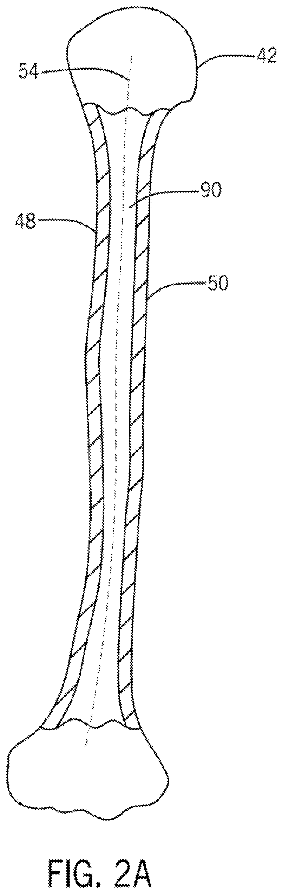

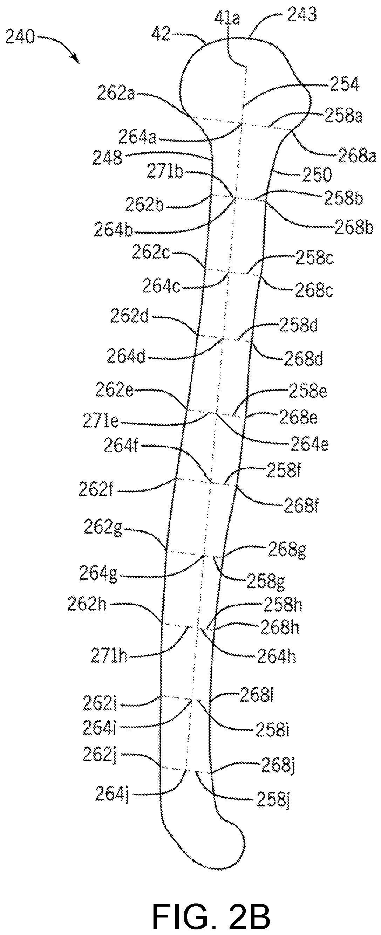

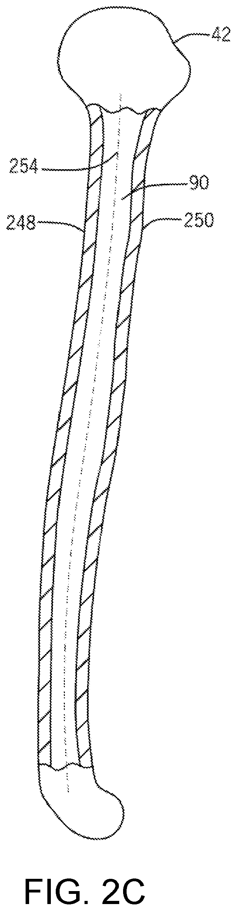

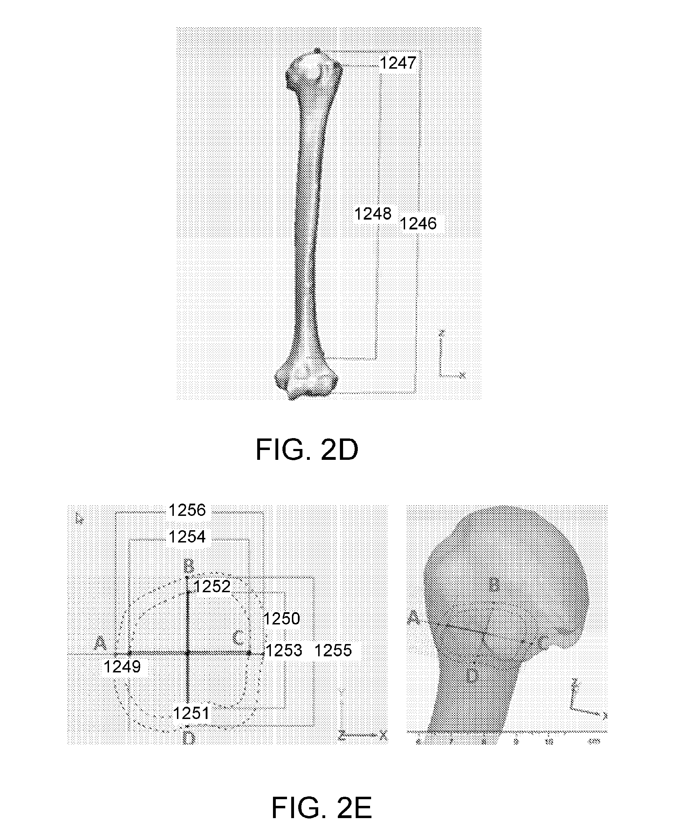

Looking at FIG. 2, the anatomic shape can be determined by a number of steps. An image 40 of a bone 42 of a subject can be obtained, in some embodiments the image 40 can be a CT image, in other embodiments the image can be an X-ray image, an ultrasonic image, a magnetic resonance image (MRI), a positron emission tomography (PET) image, or the like. The bone can be a humerus. In other embodiments the bone can be a radius, an ulna, a femur, a tibia, or any other bone. A bone cut line 46 can be oriented on the image 40 that can extend from a first border 48 of the bone 42 to an opposite second border 50 of the bone 42. In some embodiments, the bone cut line 46 can be oriented angularly across a region of a humeral head of a subject. A longitudinal bone axis 54 can be oriented on the image 40. The longitudinal bone axis 54 may extend longitudinally from a proximal humeral aspect. In another embodiment, the longitudinal bone axis 54 may extend longitudinally from an intersection 41A of a proximal aspect line, such as proximal greater tuberosity line 44 with the bone cut line 46, where the proximal greater tuberosity line 44 is oriented on the image 40 by extending perpendicularly from a first intersection point 41B on the first border 48 of the bone 42 at the most proximal and lateral aspect of a greater tuberosity through a second intersection point 41A where the proximal greater tuberosity line 44 intersects the bone cut line 46, and further extends to a third intersection point 41C on the second border 50 of the bone 42. In some embodiments, the bone 42 can be the humerus. FIG. 2A shows one embodiment where the longitudinal bone axis 54 can follow the centerline of bone 42, defined as being a constant equal distance between the first border 48 and the second border 50. When bone axis 54 is the centerline of the bone 42, the nonlinear shape of the axis line 54 defines the radius of curvature for the bone 42, which can be assessed at various points along the length of the bone axis line 54. The nonlinear shape of the axis line 54 can provide a number of different radii of curvature. When bone axis 54 is the centerline, the intersection of axis line 54 with proximal greater tuberosity line 44 may determine intersection point 41A. In one non-limiting example, a first radius of curvature can transition to a second radius of curvature, and the second radius of curvature can transition to a third radius of curvature. The first radius of curvature and the third radius of curvature can be concave, while the second radius of curvature can be convex. Each radius of curvature can feature a different radius. Any number of changes in radius of curvature can be provided such that the axis line 54 is a constant equal distance between the first border 48 and the second border 50 within the intramedullary canal 90 of the cancellous bone. In another embodiment the longitudinal bone axis 54 does not follow the centerline, but may be linear and can extend from the bone cut line 46, or may extend linearly from intersection point 41A that was established from the intersection of a centerline with proximal greater tuberosity line 44, along a length of the bone between the first border 48 and second border 50. A plurality of lateral lines 58a, 58b, 58c, 58d, 58e, 58f, 58g, 58h, 58i, 58j can be oriented on the image 40 at different distances from the intersection point 41a, or from a proximal aspect line, such as proximal greater tuberosity line 44, or from the bone cut line 46. Each of the plurality of lateral lines 58a to 58j can extend perpendicularly from one of a plurality of first intersection points 62a, 62b, 62c, 62d, 62e, 62f, 62g, 62h, 62i, 62j on the first border 48 of the bone 42 to one of a plurality of second intersection points 64a, 64b, 64c, 64d, 64e, 64f, 64g, 64h, 64i, 64j intersecting the longitudinal bone axis 54 at one of a plurality of second intersection points 64a to 64j. Each of the plurality of lateral lines 58a to 58j can further extend perpendicularly from one of a plurality of second intersection points 64a to 64j on the longitudinal bone axis 54 to one of a plurality of third intersection points 68a, 68b, 68c, 68d, 68e, 68f, 68g, 68h, 68i, 68j on the second border 50 of the bone 42. The anatomic shape of the bone 42 can be extrapolated based on determining the first intersection point 41B of the proximal greater tuberosity line 44 along with the plurality of first intersection points 62a to 62j, and measuring the distances to the corresponding second intersection points, which for intersection point 41B would be intersection point 41A of the proximal greater tuberosity line 44, and subsequently the plurality of second intersection points 64a to 64j from the first intersection points 62a to 62j. Specifically, the anatomic shape of the first border 48 of the bone 42 can be extrapolated from the first intersection point 41B of the proximal greater tuberosity line 44 and with the plurality of first intersection points 62a to 62j with the plurality of second intersection points 41a and 64a to 64j. The anatomic shape of the second border 50 of the bone 42 can be extrapolated in a similar manner as above by using the third intersection point 41C of the proximal greater tuberosity line 44 with the plurality of third intersection points 68a to 68j and measuring the distances to the corresponding second intersection points 41a and 64a to 64j.

In some embodiments, the plurality of lateral lines 58a to 58j can be placed at equidistant intervals distally from intersection point 41a, or from a proximal aspect line, such as proximal greater tuberosity line 44, or from the bone cut line 46. In some embodiments, the equidistant interval can be in a range from 0.1 to 50 millimeters. In a non-limiting embodiment, the equidistant interval can be 25 millimeters. As such, example measurements can be made at 25 millimeters, 50 millimeters, 75 millimeters, 100 millimeters, 125 millimeters, 150 millimeters, 175 millimeters, and 200 or more millimeters distal to the intersection point 41a, or from a proximal aspect line, such as proximal greater tuberosity line 44, or from the bone cut line 46. One can add more lines to provide for determining the contour of the bone with higher resolution.

In a non-limiting example embodiment, a first reference distance can be measured for a first line 71b extending perpendicularly from a first point 62b of the plurality of first intersection points 62a to 62h to a first point 64b of the plurality of second intersection points 64a to 64h. A second reference distance can be measured of a second line 71e extending perpendicularly from a second point 62e of the plurality of first intersection points 62a to 62h to a second point 64e of the plurality of second intersection points 64a to 64h. A third reference distance can be measured of a third line 71h extending perpendicularly from a third point 62h of the plurality of first intersection points 62a to 62h to a third point 64h of the plurality of second intersection points 64a to 64h.

The anatomic shape of the first border 48 can be extrapolated based on the first reference distance of the first line 71b, the second reference distance of the second line 71e, and the third reference distance of the third line 71h. A first curvature of the anatomic shape can be extrapolated between the first point 62b of the plurality of first intersection points 62a to 62h and the second point 62e of the plurality of first intersection points 62a to 62h based on the first reference distance and the second reference distance. A second curvature of the anatomic shape can be extrapolated between the second point 62e of the plurality of first intersection points 62a to 62h and the third point 62h of the plurality of first intersection points 62a to 62h based on the second reference distance and the third reference distance.

In another version of the method of the disclosure, the anatomic shape of the first border 48 and the second border 50 together can be extrapolated based on a fourth reference distance of the lateral line 58b, a fifth reference distance of the lateral line 58e, and a sixth reference distance of the lateral line 58h. A first curvature of the anatomic shape can be extrapolated between the first point 62b of the plurality of first intersection points 62a to 62j and the second point 62e of the plurality of first intersection points 62a to 62j based on the fourth reference distance and the fifth reference distance. A second curvature of the anatomic shape can be extrapolated between the second point 62e of the plurality of first intersection points 62a to 62j and the third point 62h of the plurality of first intersection points 62a to 62j based on the fifth reference distance and the sixth reference distance.

Referring to Fig. 2D, various humeral length measurements may be obtained in order to facilitate not only orthopedic plate or implant designs, but also to provide appropriate size groupings for such plates or implants to appropriately fit a patient population. In some embodiments, the overall length of the bone 1246 is determined. In the present example where the bone is the humerus, the overall length 1246 is established by creating a line between the top of the humerus and a point at the most distal portion straight down from the middle of the olecranon fossa. The distance from the top of the humerus to the top of the greater tuberosity 1247, and the distance from the greater tuberosity to the olecranon fossa 1248 may be separately recorded from the overall length 1246. In one embodiment, calculating a percentage of a patient's measured overall humeral length may be used to determine the size of plate or implant that a patient may need.

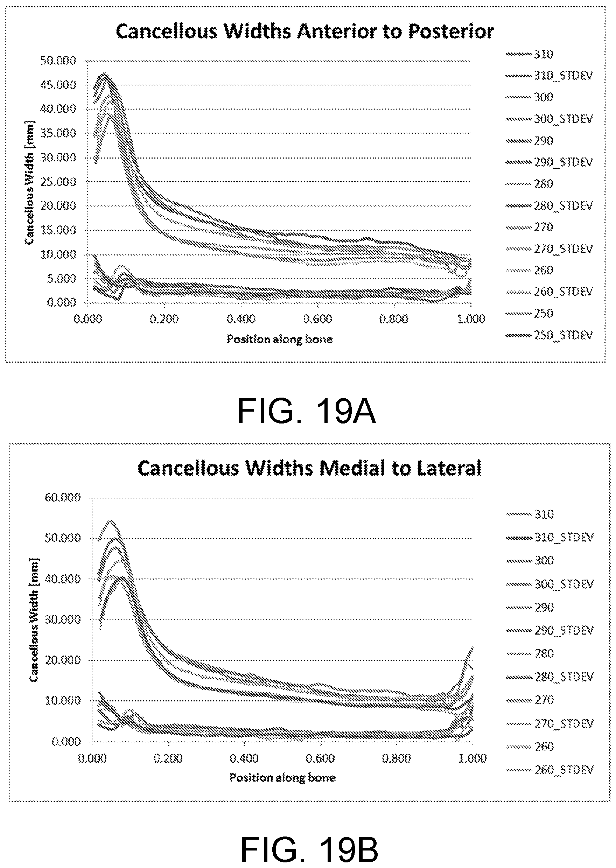

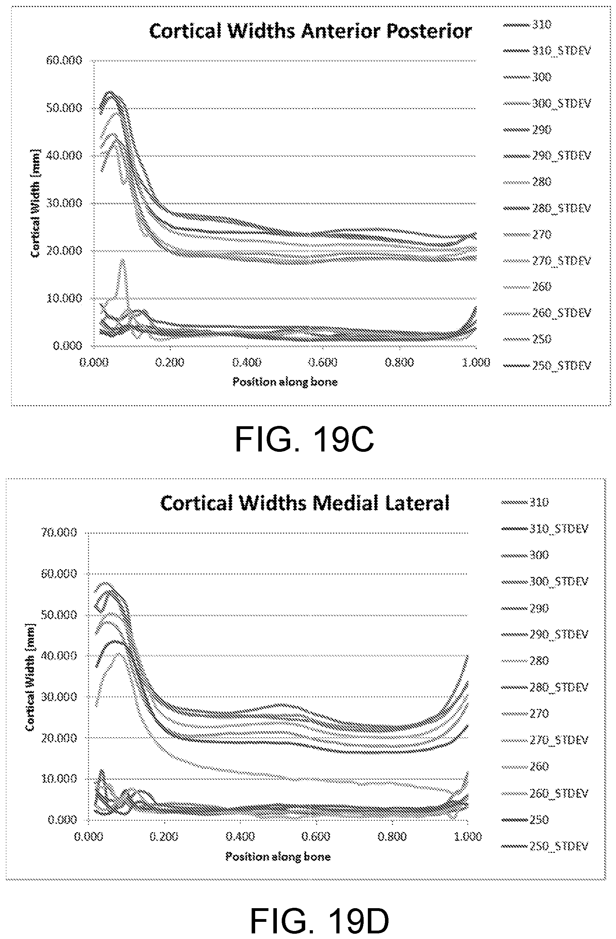

Referring to Fig. 2E, the plurality of lateral lines 58a, 58b, 58c, 58d, 58e, 58f, 58g, 58h, 58i, 58j from Fig. 2 may include being placed in multiple planes, such as A-C and D-B in Fig. 2E. The plurality of lateral lines may also include measurements of the thickness of the cortical and cancellous bone material. In the example provided, thicknesses for the cortical and cancellous bone is obtained in a 2D cross section of the humerus. Cortical lateral thickness 1249, cortical medial thickness 1250, cortical anterior thickness 1251, and cortical posterior thickness 1252 may be determined. Cancellous anterior to posterior distance 1253, cancellous medial to lateral distance 1254, cortical anterior to posterior distance 1255, and cortical medial to lateral distance 1256 may also be determined. In some embodiments, any orientation for the planes may be used, such as a partially rotated anterior to a partially rotated posterior view, which may enable for fully 3D thickness measurements of the bone. In one embodiment, these measurements may be obtained in an automated fashion, where a medical image is provided to a computer system that automatically segments the bone, identifies the relevant anatomical landmarks, such as the humeral head and the olecranon fossa, and performs the desired measurements. Graphical representations of these width measurements are shown in FIGs. 19A, 19B, 19C and 19D respectively.

Referring to FIGs. 19A, 19B, 19C, and 19D bone thickness data may be used to determine placement of a plate or implant on/in a bone. Bone thickness is a surrogate metric for bone quality, and so thickness data such as presented in the current figures may also be used by a surgeon to determine which screw holes to use for a plate during implantation. A surgeon may select screw locations to correspond to higher quality, thicker bone, and may elect to not use screws in screw hole locations that correspond to thinner bone stock. Screws, pins, bolts, cerclages, and the like may be used to attach the implant to the bone and may use the screw holes. A plate may also be repositioned on the bone in order to maximize contact with thicker bone.

The methods described above can facilitate the design of anatomically appropriate periprosthetic plates as well as mid-shaft humeral plates for the humerus with an appropriate shape, width, and length. The methods revealed a specific pattern and shape of the proximal humeral region as noted in Table 1. The greater tuberosity width was measured in the sagittal plane. The references to "anatomic cut" in Table 1 corresponds to bone cut line 46 in Fig. 2. The measurements in Table 1 are in mm and were made along longitudinal axis 54 distal to the bone cut line 46 at 25 mm (millimeters), 50 mm, 75 mm, 100 mm, 125 mm, 150 mm, 175 mm, and 200 mm along lines such as lateral lines 58a to 58h of Fig. 2, which is an example of a coronal view. The method defined contours to the humeral anatomy that would be instrumental in designing anatomically correct implants and plates, such as mid-shaft and periprosthetic plates.

**Table 1**

| Anatomic Measurements of 50 Preoperative CT Scans for Shoulder Arthroplasty | | | | | | | |
|---|---|---|---|---|---|---|---|
| **Humeral Anatomy** | Mean | Std Deviatio | Media n | Minimum | Maximum | 10th Percentile | 90th Percentile |
| Greater tuberosity width | 33.54 | 3.79 | 34.08 | 25.42 | 43.26 | 27.82 | 37.85 |
| 25 mm below anatomic cut | 29.76 | 4.27 | 29.3 | 20.8 | 37.4 | 24.3 | 35.9 |
| 50 mm below anatomic cut | 24.69 | 3.89 | 24.1 | 18.4 | 32.8 | 20.15 | 30.25 |
| 75 mm below anatomic cut | 23.15 | 3.5 | 22.65 | 16.7 | 29.9 | 18.7 | 28.6 |
| 100 mm below anatomic cut | 23.72 | 3.76 | 23.8 | 16.6 | 32.1 | 18.8 | 28.7 |
| 125 mm below anatomic cut | 22.23 | 4.01 | 21.7 | 15.5 | 31.7 | 17.5 | 26.6 |
| 150 mm below anatomic cut | 21.43 | 3.12 | 20.8 | 17 | 29.1 | 18.3 | 25.1 |
| 175 mm below anatomic cut | 22.3 | 2.94 | 22.1 | 17.6 | 30.5 | 18.2 | 26.7 |
| 200 mm below anatomic cut | 22.9 | 3.22 | 24.75 | 18.6 | 25.3 | 18.6 | 25.3 |
| The 10th and 90th percentile refer to the range of data. | | | | | | | |

As noted in Table 1, the humerus can be wide at the greater tuberosity then narrows distally until about 100 mm below the anatomic neck of the humerus. The humerus can then start to widen, which can correspond to the deltoid insertion. The humerus can then narrow until widening a second time at about 175 mm below the anatomic neck, which can correspond to the elbow region. This method and 3D modeling can facilitate developing implants and devices that can optimize contact with native bone. In one aspect, the method may be used to facilitate the minimization of soft tissue stripping, and soft tissue irritation from plates that are not contoured to the normal anatomy. Moreover, the data from this methodology can define a true anatomic distribution and range of plates needed to manage these fractures.

The methodology also revealed a significant difference in width of the humerus comparing males and females, as shown in Table 2. The anatomic cut in Table 2 corresponds to bone cut line 46 in Fig. 2. The measurements in Table 2 are in mm and were made along longitudinal axis 54 distal to the bone cut line 46 at 25 mm (millimeters), 50 mm, 75 mm, 100 mm, 125 mm, 150 mm, 175 mm, and 200 mm along lines such as lateral lines 58a to 58h of Fig. 2. One may consider having different size and shapes of humeral implants or plates for males/females as well as potentially side specific implants or plates (right and left).

**Table 2**

| Anatomic Measurements of 50 Preoperative CT Scans for Shoulder Arthroplasty Comparing Male and Female Patients | | | |
|---|---|---|---|
| **Comparison: Mean (SD)** | **Female** | **Male** | **p value** |
| Greater tuberosity width | 31.8 (2.6) | 35.1 (3.5) | 0.0015 |
| 25 mm below anatomic cut | 26.7 (2.6) | 32.6 (3.5) | <.001 |
| 50 mm below anatomic cut | 22.0 (2.5) | 27.2 (3.2) | <.001 |
| 75 mm below anatomic cut | 20.8 (2.4) | 25.3 (2.9) | <.001 |
| 100 mm below anatomic cut | 21.4 (2.8) | 25.9 (3.2) | <.001 |
| 125 mm below anatomic cut | 19.3 (2.7) | 24.9 (3.1) | <.001 |
| 150 mm below anatomic cut | 19.1 (1.5) | 23.3 (2.8) | <.001 |
| 175 mm below anatomic cut | 20.1 (1.6) | 24.1 (2.5) | <.001 |

The methodology resulted in discovery that the fracture location can be different for short compared to regular length stems. A six plate system may be employed comprising: (1) proximal humeral short periprosthetic plate, with lengths such as 85-125 mm, which may be used primarily for stemless applications or for short length stems, which have lengths such as 55-95 mm with minimal distal fracture extension; (2) proximal humeral long periprosthetic plate, with lengths such as 150-180 mm, which may be used primarily for short stems with distal fracture extension, or regular length stems, which have lengths such as 120-150 mm with minimal distal extension; (3) short distal humeral periprosthetic plate, with lengths such as 100-130 mm, which may be used primarily for short humeral stems, which have lengths such as 70-100 mm used with total elbow arthroplasty, with minimal proximal fracture extension; (4) long distal humeral periprosthetic plate, with lengths such as 150-180 mm, which may be used primarily for short humeral stems used with total elbow arthroplasty with proximal humeral fracture extension, or regular length humeral stems, which have lengths such as 120-150 mm used with total elbow arthroplasty with minimal proximal fracture extension; (5) midshaft humeral periprosthetic plate, which may be used primarily for regular length shoulder/humeral stems with minimal fracture extension or with midshaft non-periprosthetic fractures; and (6) a full-length periprosthetic plate, with lengths such as 270-330 mm for females or 300-360 mm for males, which may be used primarily for fractures that encompass a significant portion of the humerus, including highly comminuted fractures.

By understanding the fracture patterns and relative distributions, this method can then establish the distribution of plates required - short stem with a transverse fracture compared to a regular length stem with a distally extending spiral fracture. The methodology and the data set can drive accurate preoperative planning to determine the specific plate to be used at the time of surgery. This method allows for plate design that can be based upon anatomical considerations and also for taking into account fracture patterns which can then be used in preoperative planning to select the correct fracture plate needed.

To obtain plate fixation of the bone, one may consider tabs that are bendable to grab surrounding bone as well as multi-angular screws and screw holes that are adaptable to cables.

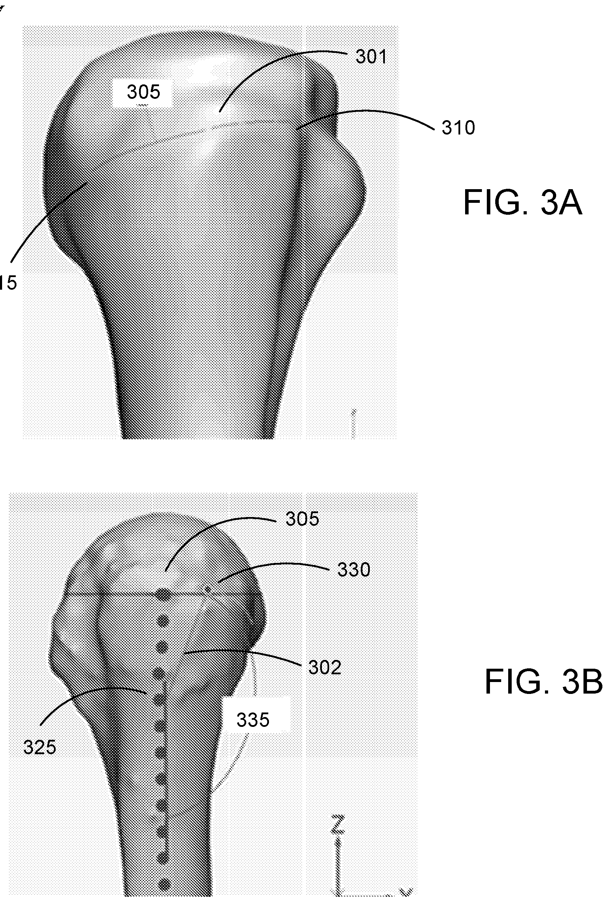

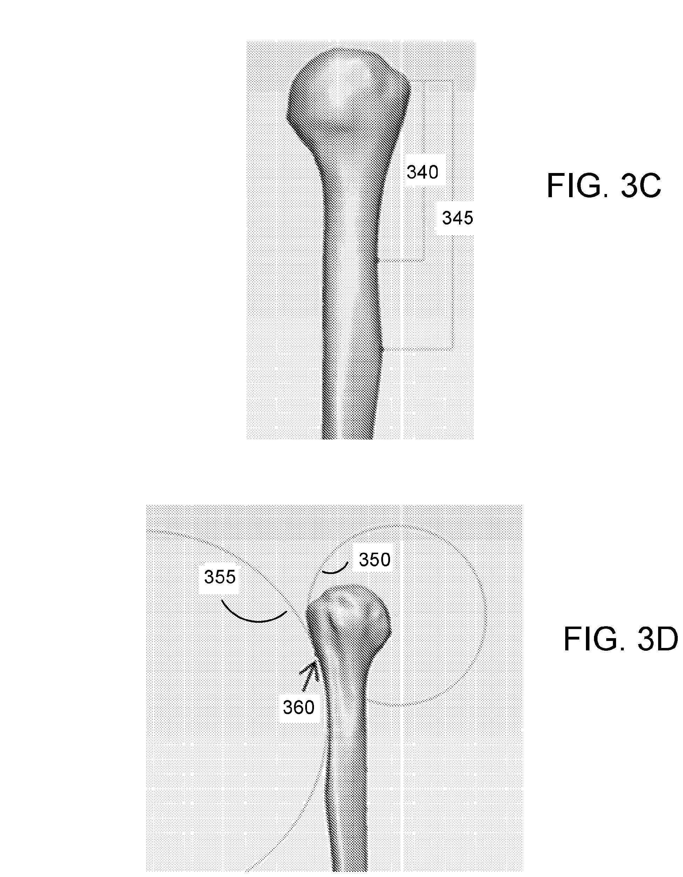

In one embodiment, the greater tuberosity (GT) width 305 was measured according to FIG. 3A in the sagittal plane by finding the most proximal and lateral point of the greater tuberosity 301 and then measuring between the most anterior border 310 to most posterior border 315 of the greater tuberosity 301. The greater tuberosity angle 335 was measured according to FIG. 3B from the centerline 325 of the cancellous bone to the mid aspect of the greater tuberosity width 330 following greater tuberosity angle line 302. The distance between the greater tuberosity to the proximal aspect of the deltoid insertion (DI) 340 was measured along with the distance from the tuberosity to the center of the deltoid insertion 345 and recorded in Table 3. FIG. 3D includes a curvature analysis where arc 350 defines the radius of curvature of the greater tuberosity on the coronal view and arc 355 defines the radius of curvatures for the region of the bone between the greater tuberosity and the deltoid insertion. The intersection point 360 is the intersection of arcs 350 and 355. The measurements in Table 3 are based upon CT scans of 50 shoulders with the data presented in mm and were made according to FIGs. 3A, 3B and 3C.

**Table 3**

| **Overall** | **GT_Width** | **GT_Angle** | **GT_to_above_DI** | **GT_to_middle_D I** |
|---|---|---|---|---|
| **Average** | 37.218 | 153.309 | 96.168 | 143.358 |
| **STDEV** | 6.123 | 7.816 | 11.197 | 14.178 |

This study was performed to further define the humeral anatomy to facilitate the design shape and size distribution of humeral implants or plates to fix fractures, such as periprosthetic fractures. A similar methodology can be used for non-periprosthetic fractures. It is also evident that implants and plates based on the true anatomy would be beneficial in other areas including hip, knee, ankle, elbow, wrist, hand, and spine including side specific implants. For example, in the femur, lines based off of the greater trochanter may be used in place of the greater tuberosity lines described here.

Various combinations of these measurements are used for manufacturing a prosthetic component, such as an implant or a plate in a subject (e.g., mammal). The prosthetic component may be formed from, for example: (i) a metal or metal alloy such as a titanium alloy (e.g., titanium-6-aluminum-4-vanadium), a cobalt alloy, a stainless steel alloy, or tantalum; (ii) a nonresorbable ceramic such as aluminum oxide or zirconia; (iii) a nonresorbable polymeric material such as polyethylene; or (iv) a nonresorbable composite material such as a carbon fiber-reinforced polymers (e.g., polysulfone), or a resorbable material, such as polyglycolic acid (PGA), and/or polylactic acid (PLA). The prosthetic component can be manufactured by machining an article formed from these materials, or by molding these materials in a suitable mold.

Looking at FIG. 2B, the anatomic shape can be determined by a number of steps. An image 240 of a bone 42 of a subject can be obtained in a sagittal viewing plane; in some embodiments the image 240 can be a CT image; in other embodiments the image can be an X-ray image, an ultrasonic image, a magnetic resonance image (MRI), a positron emission tomography (PET) image, or the like. The bone 42 can be a humerus. In other embodiments the bone can be a radius, an ulna, a femur, a tibia, or any other bone.

A longitudinal bone axis 254 can be oriented on the image 240. The longitudinal bone axis 254 may extend from intersection point 41a from FIG. 2 along a length of the bone 42 between a first border 248 and a second border 250. FIG. 2C shows one embodiment where the longitudinal bone axis 254 can follow the centerline of bone 42, defined as being a constant equal distance between the cortical bone first border 248 and the second border 250. Alternatively, longitudinal bone axis 254 may be the centerline of bone 42, being defined as a constant equal distance between the borders of the cancellous bone, which would take into account any differences with cortical bone thickness. When bone axis 254 is the centerline of the bone 42, the nonlinear shape of the axis line 254 defines the radius of curvature for the bone 42, which can be assessed at various points along the length of the bone axis line 254. The nonlinear shape of the axis line 254 can provide a number of different radii of curvature. In one non-limiting example, a first radius of curvature can transition to a second radius of curvature, and the second radius of curvature can transition to a third radius of curvature. The first radius of curvature and the third radius of curvature can be concave, while the second radius of curvature can be convex. Each radius of curvature can feature a different radius. Any number of changes in radius of curvature can be provided such that the axis line 254 is a constant equal distance between the first border 248 and the second border 250 within the intramedullary canal 90 of the cancellous bone. In one embodiment, the deviation from the straight longitudinal bone axis 254 (Fig. 2B) from the centerline following bone axis 254 (Fig. 2C) may be determined in order to indicate where over the length of the bone the area or areas of greatest bending or deflection take place. This may be used when designing plates, intramedullary nails, stems, or other implants for bends that may be needed to conform to the anatomy.

A plurality of lateral lines 258a, 258b, 258c, 258d, 258e, 258f, 258g, 258h, 258i, 258j can be oriented on the image 240 at different distances from the proximal end 243, or from point 41a. Each of the plurality of lateral lines 258a to 258j can extend perpendicularly from one of a plurality of first intersection points 262a, 262b, 262c, 262d, 262e, 262f, 262g, 262h, 262i, 262j on the first border 248 of the bone 42 to one of a plurality of second intersection points 264a, 264b, 264c, 264d, 264e, 264f, 264g, 264h, 264i, 264j intersecting the longitudinal bone axis 254 at one of a plurality of second intersection points 264a to 264j. Each of the plurality of lateral lines 258a to 258j can further extend perpendicularly from one of a plurality of second intersection points 264a to 264j on the longitudinal bone axis 254 to one of a plurality of third intersection points 268a, 268b, 268c, 268d, 268e, 268f, 268g, 268h, 268i, 268j on the second border 250 of the bone 42. The anatomic shape of the bone 42 can be extrapolated based on the plurality of first intersection points 262a to 262j, and the plurality of second intersection points 264a to 264j. Specifically, the anatomic shape of the first border 248 of the bone 42 can be extrapolated from the plurality of first intersection points 262a to 262j and the plurality of second intersection points 264a to 264j. The anatomic shape of the second border 250 of the bone 42 can be extrapolated from the plurality of second intersection points 264a to 264j and the plurality of third intersection points 268a to 268j.

In some embodiments, the plurality of lateral lines 258a to 258j can be placed at equidistant intervals distally from the proximal end 243. In some embodiments, the equidistant interval can be in a range from 0.1 to 50 millimeters. In a non-limiting embodiment, the equidistant interval can be 25 millimeters. As such, example measurements can be made at 25 millimeters, 50 millimeters, 75 millimeters, 100 millimeters, 125 millimeters, 150 millimeters, 175 millimeters, and 200 or more millimeters distal to the proximal end 243. One can add more lines to provide for determining the contour of the bone with higher resolution.

In a non-limiting example embodiment, a first reference distance can be measured for a first line 271b extending perpendicularly from a first point 262b of the plurality of first intersection points 262a to 262h to a first point 264b of the plurality of second intersection points 264a to 264h. A second reference distance can be measured of a second line 271e extending perpendicularly from a second point 262e of the plurality of first intersection points 262a to 262h to a second point 264e of the plurality of second intersection points 264a to 264h. A third reference distance can be measured of a third line 271h extending perpendicularly from a third point 262h of the plurality of first intersection points 262a to 262h to a third point 264h of the plurality of second intersection points 264a to 264h.

The anatomic shape of the first border 248 can be extrapolated based on the first reference distance of the first line 271b, the second reference distance of the second line 271e, and the third reference distance of the third line 271h. A first curvature of the anatomic shape can be extrapolated between the first point 262b of the plurality of first intersection points 262a to 262h and the second point 262e of the plurality of first intersection points 262a to 262h based on the first reference distance and the second reference distance. A second curvature of the anatomic shape can be extrapolated between the second point 262e of the plurality of first intersection points 262a to 262h and the third point 262h of the plurality of first intersection points 262a to 262h based on the second reference distance and the third reference distance.

In another version of the method of the disclosure, the anatomic shape of the first border 248 and the second border 250 together can be extrapolated based on a fourth reference distance of the lateral line 258b, a fifth reference distance of the lateral line 258e, and a sixth reference distance of the lateral line 258h. A first curvature of the anatomic shape can be extrapolated between the first point 262b of the plurality of first intersection points 262a to 262j and the second point 262e of the plurality of first intersection points 262a to 262j based on the fourth reference distance and the fifth reference distance. A second curvature of the anatomic shape can be extrapolated between the second point 262e of the plurality of first intersection points 262a to 262j and the third point 262h of the plurality of first intersection points 262a to 262j based on the fifth reference distance and the sixth reference distance.

The methods described above can facilitate the design of anatomically appropriate periprosthetic plates as well as mid-shaft humeral plates for the humerus with an appropriate shape, width, and length. The methods revealed a specific pattern and shape of the proximal humeral region as noted in Table 1.

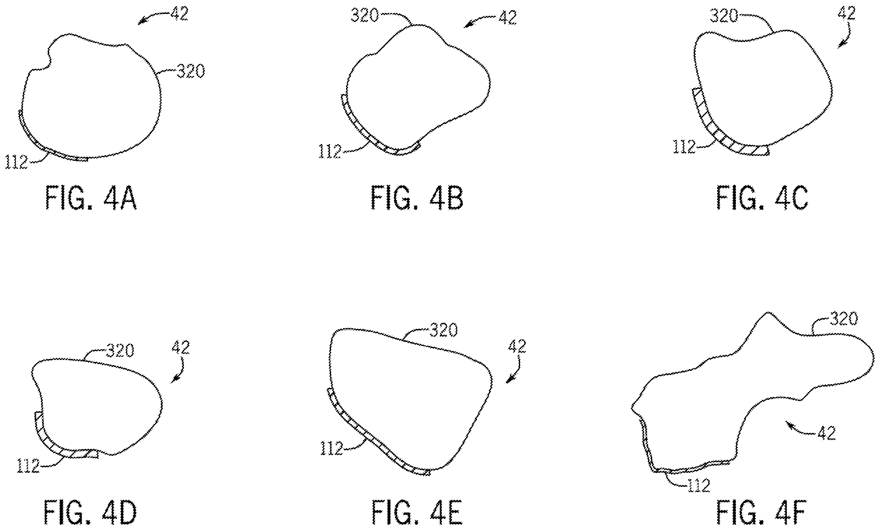

FIGs. 4A-4F show a series of traced computed tomography (CT) axial two-dimensional (2D) CT slices in axial viewing planes of the humerus along the lines 4A-4A, 4B-4B, 4C-4C, 4D-4D, 4E-4E, 4F-4F respectively in FIG. 2 with an example periprosthetic bone plate 112 installed at each location. As shown, the axial two-dimensional (2D) CT slices in axial viewing planes transition from a most proximal location in FIG. 4A to a most distal location in FIG. 4F. As shown, an outer profile 320, viewed in an axial viewing plane, of the bone 42 changes dramatically from FIG. 4A to FIG. 4F (i.e. proximal to distal). Lines 4A-4A, 4B-4B, 4C-4C, 4D-4D, 4E-4E, and 4F-4F each have corresponding planes P4A, P4B, P4C, P4D, P4E, and P4F respectively extending outwardly along the same path of lines 4A-4A, 4B-4B, 4C-4C, 4D-4D, 4E-4E, and 4F-4F respectively as depicted in FIG. 2.

One of skill in the art will appreciate that although FIGs. 4A-4F show the bone plate 112 present at each axial location, the bone plate 112 may be present in any of FIGs. 4A-4F, and may also not be present in any of FIGs. 4A-4F. The bone plate 112 can further be in some but not all views FIGs. 4A-4F. A non-limiting example could include the bone plate 112 in FIGs. 4A to 4C, and not include the bone plate 112 in FIGs. 4D to 4F. The bone plate 112 may also appear in a different location in the views or between the views of FIGs. 4A-4F due to the curvature of the plate and the desired location in regard to the associated anatomy.

The methods described above can facilitate the design of anatomically appropriate periprosthetic plates as well as mid-shaft humeral plates for the humerus with an appropriate shape, width, and length. This method can be automated where images are sent to a control system having a processor configured to execute a program stored thereon to automatically extract measurements of the bone 42 of the subject. An automated system may use machine learning routines to perform the measurements or to analyze the measurements for the design of plates or implants. The measurements of the bone 42 can be referenced to manufacture a 3D plate or implant that is created for a specific patient. In some embodiments, the 3D plate can be three-dimensionally manufactured using an additive manufacturing system. In some embodiments, the plate can be metal. A surgeon can then implant this patient specific implant/plate. For fracture applications, image data from an opposite extremity, e.g. an opposite, arm can be mirrored to create the plate/implant for the repair. For an implant, the stem can be similarly designed to fit the interior aspect of the bone rather than forcing a uniform cylinder into a patient's humerus.

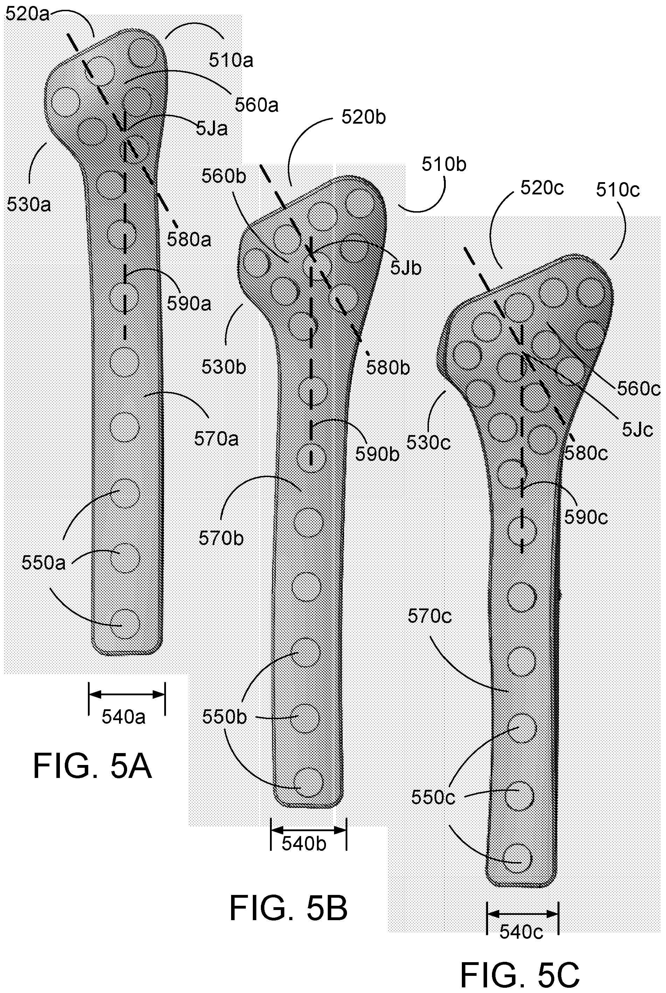

In one embodiment, a detailed analysis of the measurements provides for developing three different size models of proximal humeral plates. Referring particularly to FIGs. 5A, 5B and 5C, example proximal humeral anatomy plates are shown in three different sizes. Fig. 5A depicts the smallest size, Fig. 5B depicts a medium size, and Fig. 5C depicts the largest size. The models were created to test the methodology. The methodology resulted in a model that is left and right side specific. The curvature of the superior aspect of the plates 510a, 510b, and 510c was designed to match the curvature of the anatomy at the location of the greater tuberosity near the bicipital groove in such a way as to maximize contact between the plate and the bone and to prevent interference or pinching of soft tissue when a subject implanted with such a plate raises their arm. Sloped flat portion 520a, 520b, and 520c follows the anatomy of the greater tuberosity where the greater tuberosity angle was developed so that the distal aspect of the plate sits more in line with the anterior humeral shaft, thereby decreasing the need for deltoid stripping and soft tissue detachment. Curvature 530a, 530b, and 530c blends the proximal plate portion 560a, 560b, and 560c with the distally extending plate portion 570a, 570b, and 570c that extends down the humerus towards the deltoid insertion. Proximal plate portion 560a, 560b, and 560c includes longitudinal axis 580a, 580b, and 580c, and distal extending plate portion 570a, 570b, and 570c includes longitudinal axis 590a, 590b, and 590c. In one configuration, axis 590a, 590b, and 590c intersects axis 580a, 580b, and 580c at junction 5Ja, 5Jb, and 5Jc, and the angle between proximal plate portion axis 580a, 580b, and 580c and distal plate portion axis 590a, 590b, and 590c is determined by "GT Angle" noted in Table 3. One aspect of the models was the ability to maximize proximal humeral fixation with a plate that is anatomic in shape and in the appropriate size distribution.

An important aspect of fixation of proximal humerus fractures is related to the ability to maximize fixation in the proximal humeral region corresponding to proximal plate portion 560a, 560b, and 570c. Holes for providing attachment to the bone include screw holes 550a, 550b, and 550c which aid in providing fixation between the plate and the bone. Screws, pins, bolts, cerclage, and the like may be used to attach the plate to the bone and may use the screw holes 550a, 550b, 550c. It will be appreciated that different numbers and sizes of screw holes could be used depending upon the desired amount of fixation and hardware available. All currently available plates only have one size plate width for the proximal humeral region. This is the area where fracture fixation most commonly fails. In addition, this is the region where non-anatomic plates contribute to malposition of the fragments as well as the need for more soft tissue disruption to place the plates. The proximal shape of the models can be extended a variable distance distally down the shaft, corresponding to distally extending plate portion 570a, 570b, and 570c, based on the length of fracture propagation distally and the length of the plate desired by the company or surgeon. Width 540a, 540b, and 540c may also be selected to address a distal fracture.

In one embodiment, the anterior to posterior slope 520a, 520b, and 520c of the most proximal aspect of the plate was designed based on the natural curvature of the greater tuberosity. This anatomic shape optimizes plate-bone contact, maximizes the ability of the plate to cradle and support the proximal humeral region, increases the number of proximal screws to improve fixation while at the same time minimize impingement on the acromion with shoulder elevation. In FIGs. 5A-C, each of the three size plates has an optimized number of screw holes 550a, 550b, and 550c located proximally to maximize fixation in the appropriately sized patient. FIG. 5A features 3 screw holes 550a at the most superior aspect, while FIG. 5B features 4 screw holes 550b and FIG. 5C features 5 screw holes 550c. A user has flexibility in choosing the specific screw holes 550a, 550b, and 550c utilized. The three examples shown in FIGs. 5A-C highlight the benefits of the methodology and the potential to maximize fixation and tailor to different size patients.

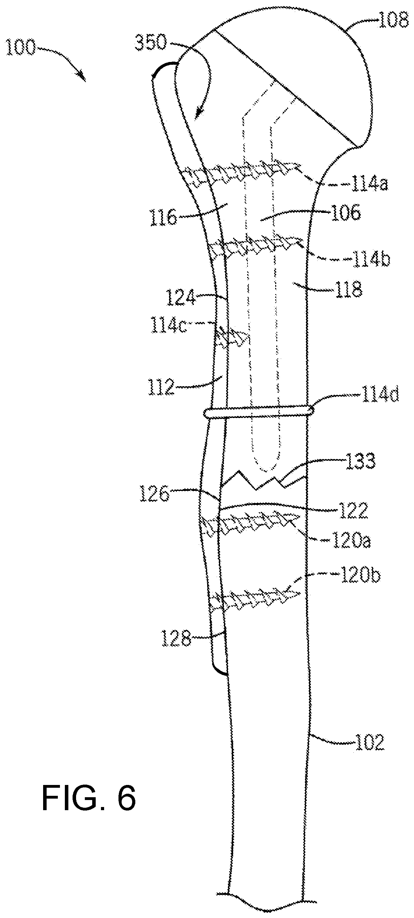

In one embodiment, the methodology also resulted in defining the arcs of curvature of the proximal humeral region improving the ability of the plate to be appropriately placed on the bone minimizing the need for plate bending and importantly, malpositioning the fracture in regard to angulation. This can be seen in FIG. 3D. FIG. 6 shows one non-limiting embodiment of a periprosthetic bone plate 112 that can be designed using the method of the disclosure viewed in an anterior-posterior (AP) viewing plane. An example periprosthetic treatment system 100 is shown in a bone 102 having a fracture 133. The periprosthetic treatment system 100 includes a stem 106 that extends into a bore formed within the bone 102. The bone 102 can be the humerus. Typically, the stem 106 is fixed within the bore formed within the bone 102. A generally hemispherical head 108 is connected to the stem 106. The stem 106 can be monolithic with the head 108, or the stem 106 and the head 108 can be formed as separate parts. The hemispherical head 108 can articulate with a complementary concave section of a glenoid component that is fixed within the glenoid cavity of the scapula using cemented or uncemented posts. One skilled in the art would appreciate that other prostheses, such as reverse shoulder arthroplasty, hemi arthroplasty, stemless shoulder arthroplasty, resurfacing, elbow prostheses and the like may be suitable for use with example bone plate 112.

The periprosthetic treatment system 100 further includes a periprosthetic bone plate 112. The periprosthetic bone plate 112 may be formed according to any of the methods described above such that the periprosthetic bone plate 112 is formed to conform to the anatomy of the subject. The periprosthetic bone plate 112 may further include openings for receiving one or more periprosthetic fasteners 114a-114d. The one or more periprosthetic fasteners 114a-114d can extend through the periprosthetic bone plate 112, through a first portion 116 of the bone 102, to the side of the stem 106, and may extend into a second portion 118 of the bone 102. The one or more periprosthetic fasteners 114a-114d may also be short enough to only extend into a first portion 116 of the bone 102. In some embodiments, at least one of the one or more periprosthetic fasteners 114a-114d may also be a cable that extends around the periprosthetic bone plate 112 and the bone 102. As shown, the periprosthetic fasteners 114 are configured to secure the periprosthetic bone plate 112 to the bone 102. The periprosthetic treatment system 100 may also include one or more fasteners 120a, 120b that extend through the periprosthetic bone plate 112 into the bone 102. In some non-limiting embodiments, the one or more fasteners 120a, 120b can be positioned distally to the one or more periprosthetic fasteners 114a-114d. In other embodiments, the fasteners 120a, 120b can be positioned proximally to the periprosthetic fasteners 114a-114d. The periprosthetic fasteners 114a-114d and the fasteners 120a, 120b can be any appropriate mechanical fastening elements, for example, bone screws, wires, cables etc.

As shown, the periprosthetic plate 112 is designed to be anatomically correct for the bone depicted. A bone interface surface 122 of the periprosthetic plate 112 at a proximal portion 124 of the periprosthetic plate 112 may be convex to accommodate the shape of the bone 102. The bone interface surface 122 can transition from convex at the proximal portion 124 to be concave at an intermediate portion 126 of the periprosthetic plate 112 to accommodate the shape of the bone. The bone interface surface 122 can transition from concave at the intermediate portion 126 to be convex at a distal portion 128 of the periprosthetic plate 112 to accommodate the shape of the bone. In one embodiment, periprosthetic plate 112 is designed to be anatomically correct using the curvature analysis information according to FIG 3D to maximize contact between the bone 102 and the plate 112 and thereby minimizing the bone interface surface 122.

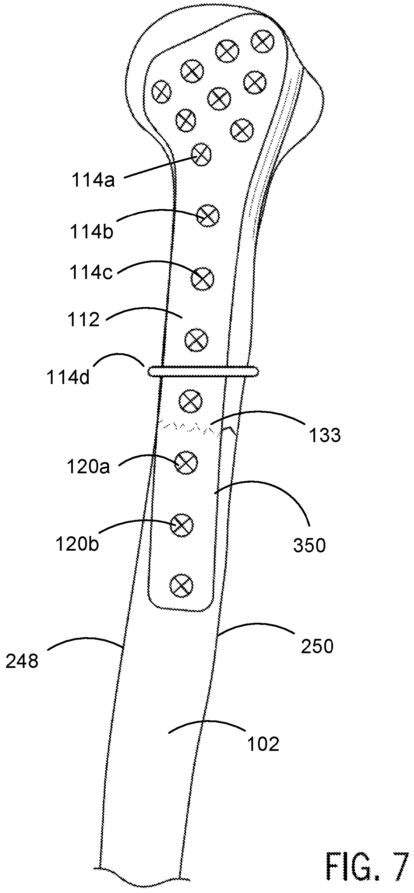

FIG. 7 shows the non-limiting embodiment of a periprosthetic bone plate 112 that can be designed using the method of the disclosure of FIG. 6 viewed in a sagittal viewing plane.

Looking at FIGs. 6 to 8, the periprosthetic bone plate 112 can be contoured in multiple degrees of freedom. Specifically, the three-dimensional (3D) anatomy of the bone 42 of the subject is taken into account with the contours of the periprosthetic bone plate 112 as well as the relevant soft-tissue anatomy. The periprosthetic bone plate 112 may be contoured in multiple degrees of freedom. Non limiting examples of shapes that can characterize the periprosthetic bone plate include spiral, double curve, bone wrapping/hugging, and others. Accordingly, the periprosthetic bone plate 112 can have varying thickness at different locations along the plate (see also FIGs. 4A-4F). In some embodiments, it may be desirable to have a greater thickness of a periprosthetic bone plate generally for male subjects and a generally thinner thickness periprosthetic bone plate for female subjects.

In some embodiments the periprosthetic plate 112 can be an elongated plate dimensioned for placement on the bone 42 across the fracture 133, the periprosthetic plate 112 having a bone interface surface 350 that faces the bone 42 when the plate 112 is placed on the bone 42 across the fracture 133.

In a non-limiting embodiment, the bone interface surface 350 can have a proximal region proximal to a first plane transverse to the elongated plate (e.g., P4C as shown in FIG. 2), a distal region distal to a second plane transverse to the elongated plate (e.g., P4D as shown in FIG. 2), and a midshaft region positioned between the first plane (e.g., P4C) and the second plane (e.g., P4D).

The bone interface surface 350 can have a shape that transitions from a first curvature that is convex or concave at a proximal portion of the plate 112 to a second curvature at a second portion of the plate 112 longitudinally adjacent to the proximal portion of the plate. The second curvature can be convex when the first curvature is concave, and the second curvature being concave when the first curvature is convex. In some embodiments, the first curvature can be convex. The shape of the bone interface surface 350 can transition from the second curvature to a third curvature at a distal portion of the plate 112 longitudinally adjacent to the second portion of the plate 112, the third curvature being convex when the second curvature is concave, and the third curvature being concave when the second curvature is convex.

The proximal region of the bone interface surface 350 can conform to a bone surface proximal to the first plane (e.g., P4C). The distal region of the bone interface surface 350 can conform to a bone surface distal to the second plane (e.g., P4D). The midshaft region of the bone interface surface 350 can conform to a bone surface between the first plane (e.g., P4C) and the second plane (e.g., P4D). A second proximal region of the bone interface surface 350 can conform to a bone surface proximal to the first plane (e.g., P4C) and distal to the proximal region (e.g., between P4B and P4C). A second distal region of the bone interface surface 350 can conform to a bone surface distal to the second plane (e.g., P4D) and proximal to the distal region (e.g., between P4D and P4E). As such, the proximal region can be positioned proximal to the second proximal region (e.g., between P4A and P4B), and the distal region can be positioned distal to the second distal region (e.g., between P4E and P4F).

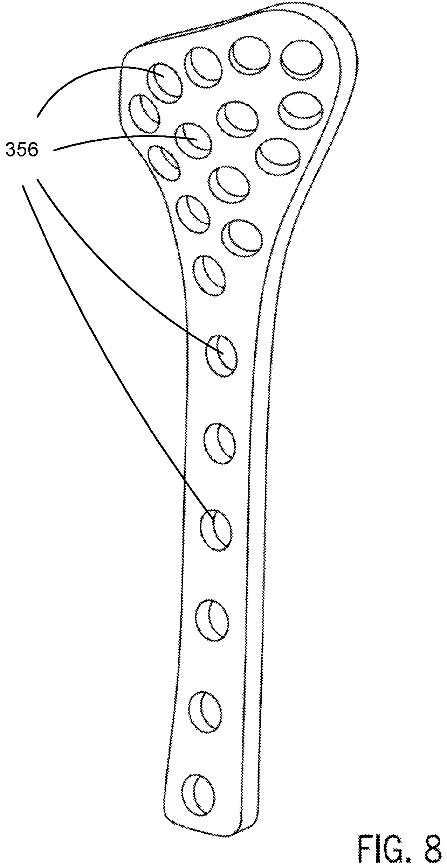

In some embodiments, the plate 112 can include a plurality of openings 356 for receiving bone engaging fasteners (periprosthetic fasteners 114a-114d) therethrough. The plate 112 can be a proximal humeral short periprosthetic plate formed for a short stem of the prosthesis with a minimal distal fracture extension. Additional openings for receiving bone engaging fasteners are depicted in FIG. 8.

In some embodiments, the plate 112 can be a proximal humeral long periprosthetic plate formed for at least one of short stems of the prosthesis with a distal fracture extension and a regular length stem of the prosthesis with a minimal distal extension.

In some embodiments, the plate 112 can be a short distal humeral periprosthetic plate formed for a short humeral stem of the prosthesis used with a total elbow arthroplasty, with a minimal proximal fracture extension.

In some embodiments, the plate 112 can be a long distal humeral periprosthetic plate, formed for a short humeral stem of the prosthesis used with at least one of a total elbow arthroplasty with a proximal humeral fracture extension and a regular length humeral stem of the prosthesis used with a total elbow arthroplasty with a minimal proximal fracture extension.

In some embodiments, the plate 112 can be a midshaft humeral periprosthetic plate formed for at least one of a regular length shoulder/humeral stem of the prosthesis with a minimal fracture extension and a midshaft non-periprosthetic fracture.

In some embodiments, the plate 112 can be a full-length periprosthetic plate formed for fractures that encompass a significant portion of the humerus, including highly comminuted fractures.

In some embodiments, a device for treating a fracture between a proximal section of a bone and a distal section of the bone is provided. The device can comprisea prosthesis 100 configured to be implanted in a bone 42; and an elongated plate 112 dimensioned for placement on the bone 42 across the fracture 133. The plate 112 can have a bone interface surface 350 that faces the bone 42 when the plate 112 is placed on the bone 42 across the fracture 133. The bone interface surface 350 can have a shape that transitions from a first curvature that is convex or concave at a proximal portion of the plate 112 to a second curvature at a second portion of the plate 112 longitudinally adjacent to the proximal portion of the plate 112, the second curvature being convex when the first curvature is concave, and the second curvature being concave when the first curvature is convex.

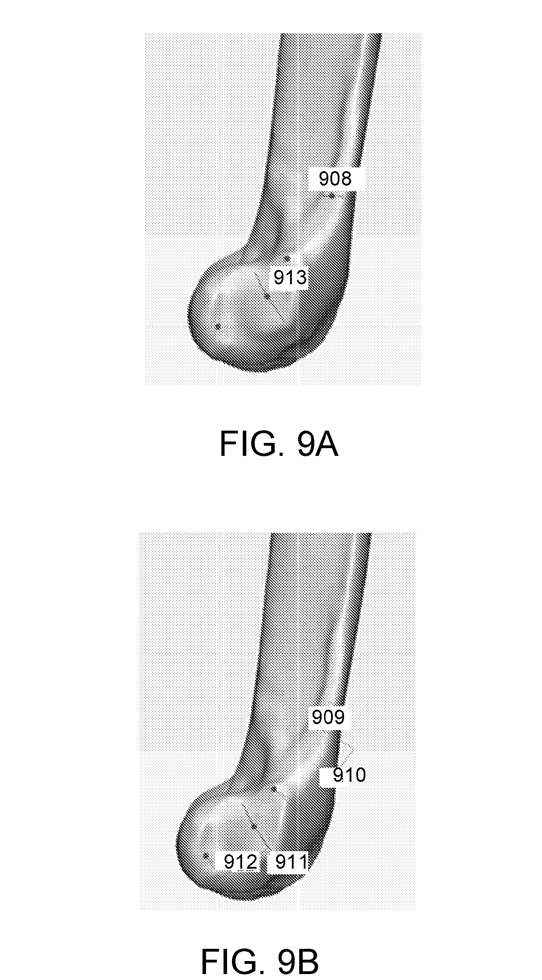

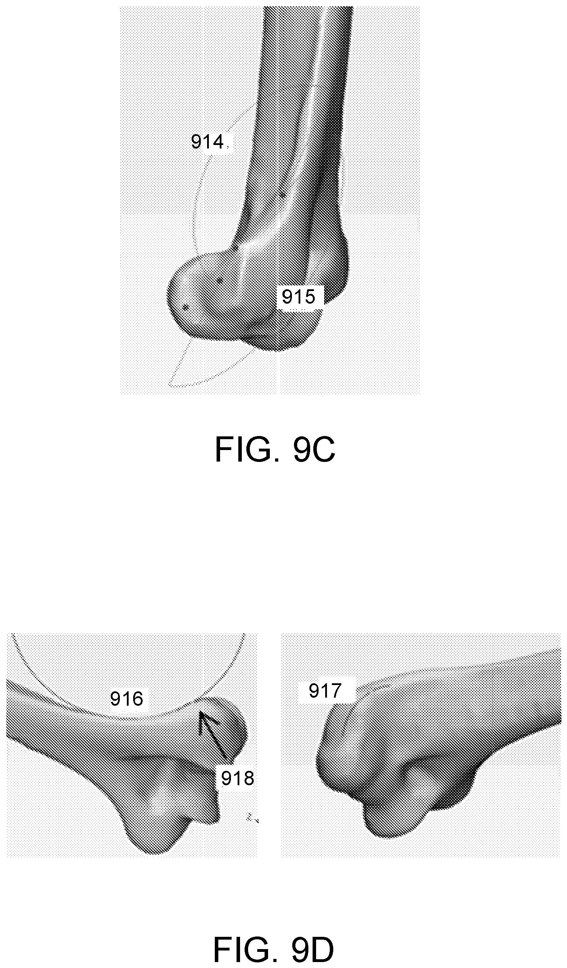

Referring to Figs. 9A-9D, the method may be applied to the distal humerus at the lateral condyle. One skilled in the art will understand that the measurement provided here can be obtained in any order and not all measurements may be needed depending upon the device being designed or the intervention being considered. In Fig. 9A, first lateral condyle width 908 is drawn at the level of the olecranon fossa in a sagittal view as shown, and the maximum width 913 of the lateral condyle is determined. In Fig. 9B, a first length line 909 is created to be perpendicular to width 908 and extends to the cancellous centerline of the humerus. The location of the most proximal aspect of the lateral epicondyle is established and second length 910 is created between this point and the center of width line 908. The location of the most distal aspect of the lateral humerus is established and a third length 911 is established to connect this point with the most proximal aspect of the lateral epicondyle. Fourth length 912 is established to measure the length from the midpoint of width 913 to the most distal aspect of the lateral humerus. In Fig. 9C, the first angle of the lateral condyle 914 is determined by using the point at the most proximal aspect of the lateral epicondyle, to the midpoint of first width 908, and to the lateral edge of the humeral shaft. The second angle of the distal portion of the lateral condyle 915 is established using first width 908 midpoint to the lateral epicondyle point, and to the maximum width 913 midpoint. In Fig. 9D, the radii of curvature for the lateral condyle are determined. The first radius of curvature 916 for the lateral condyle is established by creating an arc following the curvature of the lateral condyle to the lateral epicondyle on the coronal view. The second radius of curvature 917 is established by creating an arc following the curvature starting from the lateral epicondyle to the most distal aspect of the condyle. Intersection point 918 is determined by locating the intersection between the first radius of curvature 916 and the second radius of curvature 917. A summary of lateral condyle data is provided with Table 4.

**Table 4**

| | **OVERALL** | | | | | |
|---|---|---|---|---|---|---|
| | **LC Max Width** | **LC_Width 1** | **LC_Length_ 1** | **LC_Length_** | **LC_Length_ 3** | **LC_Length_ 4** |
| **Averag e** | 11.423 | 5.011 | 20.457 | 17.336 | 18.908 | 10.989 |
| **STDEV** | 2.099 | 1.097 | 7.490 | 4.567 | 3.363 | 2.424 |
| | | | | | | |

| | **LC_Angle_1** | **LC_Angle_ 2** | | | | |
|---|---|---|---|---|---|---|
| **Averag e** | 158.485 | 147.034 | | | | |
| **STDEV** | 7.184 | 7.636 | | | | |

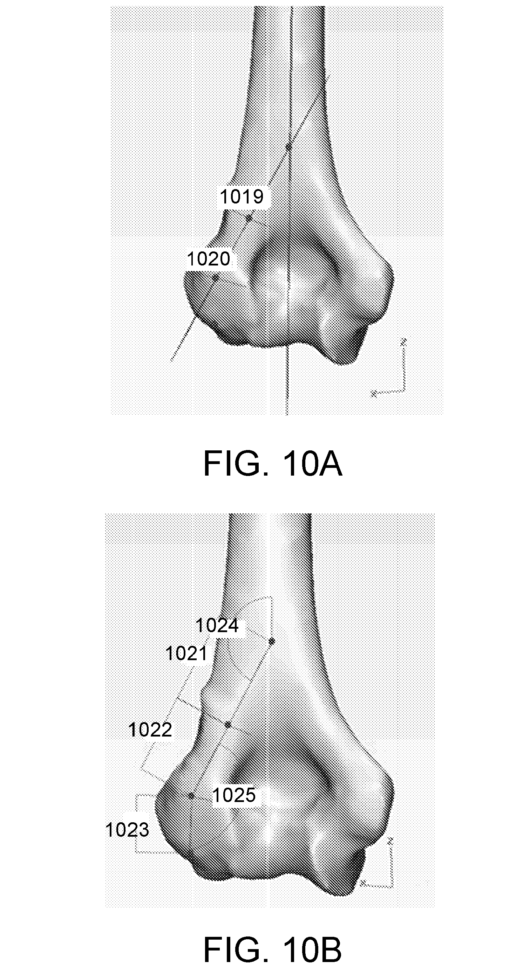

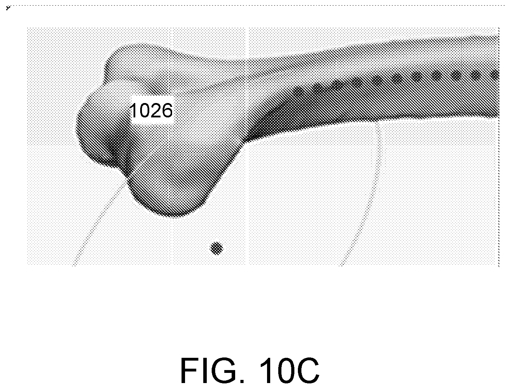

Referring to Figs. 10A-10C, the method may be applied to the distal humerus at the posterior lateral condyle. In Fig. 10A, first width 1019 is established by creating a line at the surface from the most lateral to the most medial surface of the posterior lateral condyle perpendicular to the centerline of the posterior lateral condyle on the coronal view at the level of the olecranon fossa. A maximum width 1020 is established by creating a line at the greatest width of the posterior lateral condyle. In Fig. 10B, a first length 1021 is established by creating a line that connects the centerline of the cancellous bone to the midpoint of first width line 1019. A second length 1022 is established by measuring the length from the midpoints of the lines of first width 1019 to maximum width 1020. A third length 1023 is established by locating a point straight down (down z axis) from the midpoint of maximum width 1020 to the most distal end of the posterior lateral humerus at the edge of the bone. A first angle 1024 of the posterior lateral condyle is established by creating an angle from width 1019 to the most proximal point of third length 1023 to a point on the cancellous centerline. A second angle 1025 of the posterior lateral condyle is established by creating an angle from width 1019 midpoint to maximum width 1020 midpoint to the most distal end of the posterior lateral humerus at the edge of the bone. In Fig. 10C, the radius of curvature 1026 is established by using a sagittal view and creating an arc on the posterior side following the curve of the posterior lateral condyle. A summary of posterolateral condyle (PLC) data is provided with Table 5, wherein STDEV is the standard deviation of the data.

**Table 5**

| | **OVERALL** | | | | |
|---|---|---|---|---|---|
| | **PLC Max Width** | **PLC_Width_ 1** | **PLC_Length_ 1** | **PLC_Length_ 2** | **PLC_Length_ 3** |
| **Average** | 19.612 | 13.144 | 34.873 | 19.410 | 9.961 |
| **STDEV** | 3.023 | 2.400 | 13.653 | 3.320 | 2.255 |
| | | | | | |

| | **PLC_Angle_1** | **PLC_Angle_ 2** | | | |
|---|---|---|---|---|---|
| **Average** | 152.441 | 154.313 | | | |
| **STDEV** | 21.746 | 5.269 | | | |

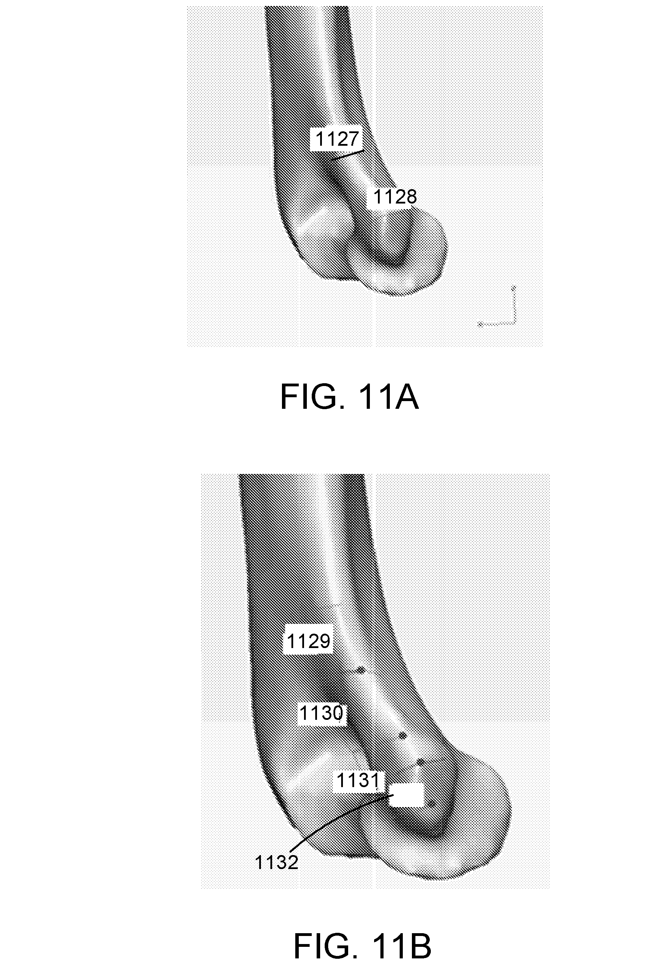

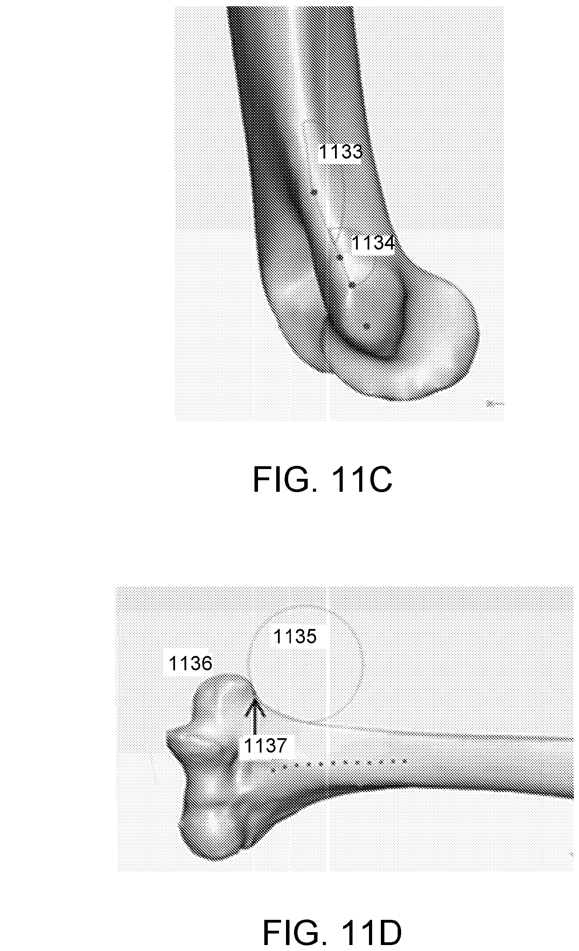

Referring to Figs. 11A-11D, the method may be applied to the distal humerus at the medial condyle (MC). In Fig. 11A, first width 1127 is established by creating a line from the most anterior to posterior borders of the medial condyle at the level of the olecranon fossa on a sagittal view. Maximum width 1128 is established by measuring the greatest width of the medial condyle. In Fig. 11B, first length 1129 is established by creating a line perpendicular to width 1127 that extends from the midpoint of width 1127 to the cancellous centerline of the humerus. Second length 1130 is established by determining the length from width 1127 midpoint to the most proximal aspect of the medial epicondyle. Third length 1131 is established by determining the length from the point of the proximal aspect of the medial epicondyle to the most distal end of the humerus. Fourth length 1132 is established by determining the length from maximum width 1128 midpoint to the most distal end of the humerus. In Fig. 11C, first angle 1133 of the medial condyle is created by using the point at the most proximal aspect of the medial epicondyle to the midpoint of first width 1127 and to the medial edge of the shaft. Second angle 1134 is determined using first width 1127 midpoint to the medial epicondyle point to the maximum width 1128 midpoint. In Fig. 11D, the radii of curvature for the medial condyle are determined. First radius of curvature 1135 is determined by creating an arc following the curvature of the medial condyle to the medial epicondyle on the coronal view. Second radius of curvature 1136 is established by creating an arc following the curvature starting from the medial epicondyle to the most distal aspect of the humerus. Intersection point 1137 is determined by finding where the first radius of curvature 1135 intersects with the second radius of curvature 1136. A summary of medial condyle data is provided with Table 6, wherein STDEV is the standard deviation of the data.

**Table 6**

| | **OVERALL** | | | | | |
|---|---|---|---|---|---|---|
| | **MC Max Width** | **MC_Width_1** | **MC_Length_1** | **MC_Length_2** | **MC_Length_3** | **MC_Length_4** |
| **Average** | 11.654 | 7.262 | 13.184 | 16.891 | 13.418 | 6.714 |
| **STDEV** | 2.418 | 2.071 | 7.862 | 3.803 | 2.407 | 1.861 |
| | | | | | | |

| | **MC_Angle_1** | **MC_Angle_2** | | | | |
|---|---|---|---|---|---|---|
| **Average** | 159.093 | 174.549 | | | | |
| **STDEV** | 5.967 | 4.580 | | | | |

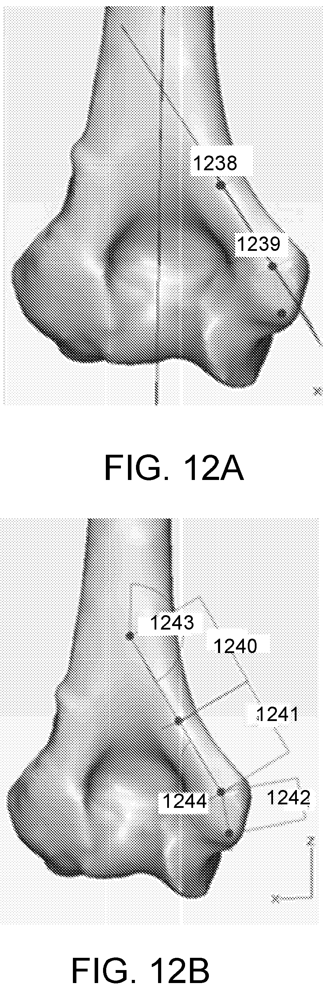

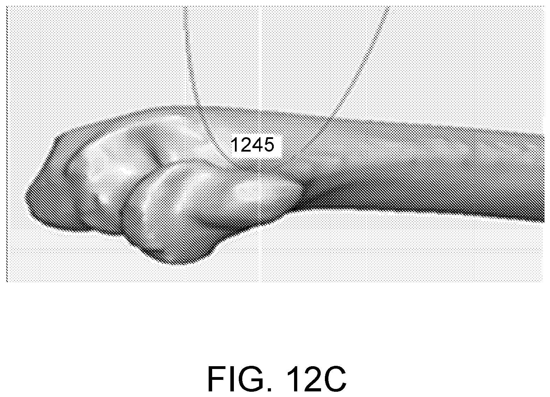

Referring to Figs. 12A-12C, the method may be applied to the distal humerus at the posterior medial condyle. In Fig. 12A, first width 1238 is established by creating a line from the most lateral to the most medial surface of the posterior medial condyle, perpendicular to a centerline of the posterior medial condyle on the coronal view at the level of the olecranon fossa. The maximum width 1239 is determined by creating a line at the greatest width of the posterior medial condyle parallel with first width 1238. In Fig. 12B, first length 1240 is determined by creating a line connecting the center line of the cancellous bone to the midpoint of first width 1238. Second length 1241 is determined by finding the length from the first width 1238 midpoint to the maximum width 1239 midpoint. Third length 1242 is established by creating a line connecting a point straight down (down z axis) from the midpoint of maximum width 1239 to the most distal end of the posterior medial humerus. First angle 1243 of the posterior medial condyle is established by creating an angle between the centerline of the posterior medial condyle and the centerline of the cancellous humerus. Second angle 1244 of the distal portion of the posterior medial condyle is established by creating an angle from the first width 1238 midpoint to the maximum width 1239 midpoint to the point straight down (down z axis) at the most distal end of the posterior medial humerus. In Fig. 12C, the radius of curvature 1245 for the posterior medial condyle is established using a sagittal view and creating an arc on the posterior side following the curve of the posterior medial condyle. A summary of posteromedial condyle (PMC) data is provided with Table 7, wherein STDEV is the standard deviation of the data.

**Table 7**

| | **OVERALL** | | | | |
|---|---|---|---|---|---|
| | **PMC Max Width** | **PMC_Width_ 1** | **PMC_Length_ 1** | **PMC_Length_ 2** | **PMC_Length_ 3** |
| **Average** | 13.716 | 9.813 | 28.907 | 19.213 | 7.676 |
| **STDEV** | 2.299 | 1.835 | 7.552 | 2.828 | 1.886 |

| | **PMC_Angle_ 1** | **PMC_Angle_ 2** | | | |
|---|---|---|---|---|---|
| **Average** | 147.673 | 148.682 | | | |
| **STDEV** | 6.097 | 4.211 | | | |

In one embodiment, a compilation of data may be used to aid in the design of plate sizes by indicating patient size trends. Table 8 provides a compilation of the data discussed above along with statistical percentiles, wherein STDEV is the standard deviation of the data.

**Table 8**

| | ***10^{th} Percentile [mm]*** | ***50^{th} Percentile [mm]*** | ***90^{th} Percentile [mm]*** | ***Minimum [mm]*** | ***Maximum [mm]*** | ***Mean*** | ***STDEV*** |
|---|---|---|---|---|---|---|---|
| ***GT Width*** | 29.110 | 36.258 | 46.152 | 25.570 | 52.043 | 37.218 | 6.123 |
| ***LC Width 2*** | 8.807 | 11.075 | 14.534 | 7.623 | 15.575 | 11.423 | 2.099 |
| ***PLC Width 2*** | 14.948 | 19.751 | 23.236 | 12.339 | 25.995 | 19.612 | 3.023 |
| ***MC Width 2*** | 8.303 | 11.408 | 14.922 | 6.422 | 16.310 | 11.654 | 2.418 |
| ***PMC Width 2*** | 11.005 | 13.473 | 16.495 | 8.985 | 19.886 | 13.716 | 2.299 |
| ***Full Length*** | 289.529 | 319.633 | 349.584 | 280.400 | 362.202 | 322.63 6 | 21.570 |

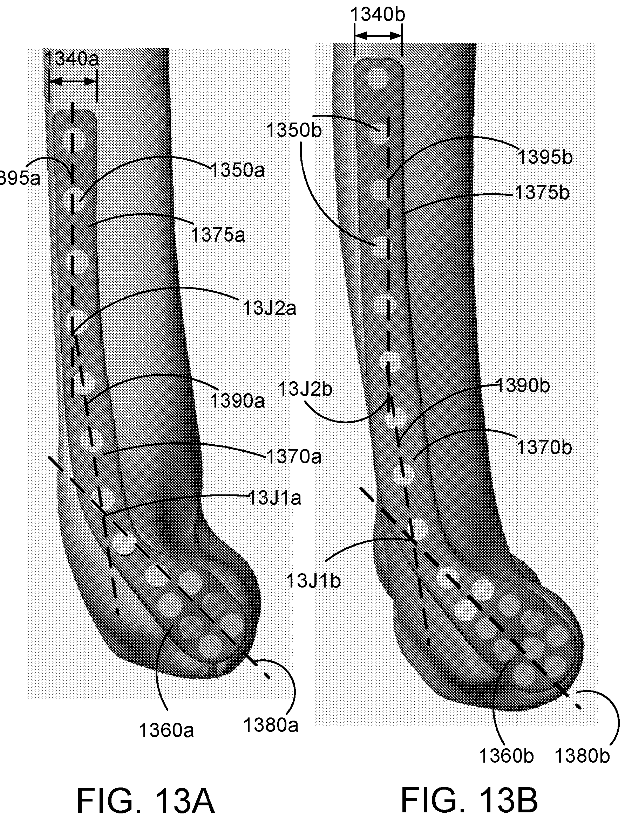

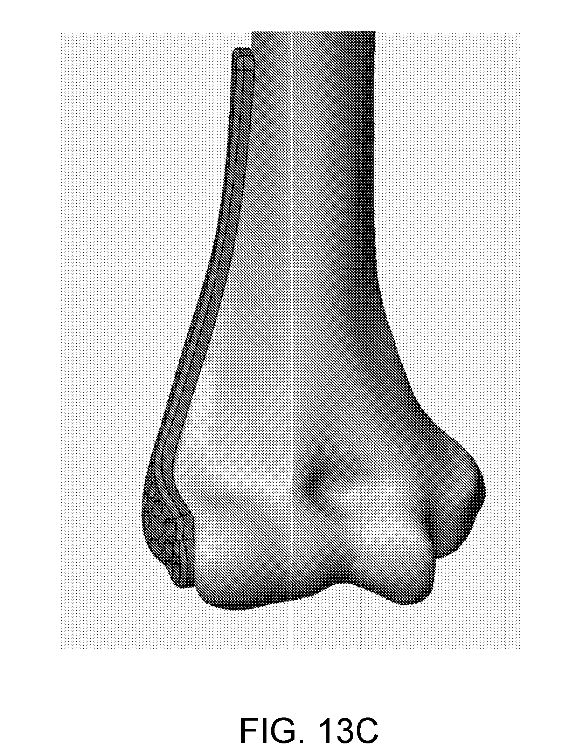

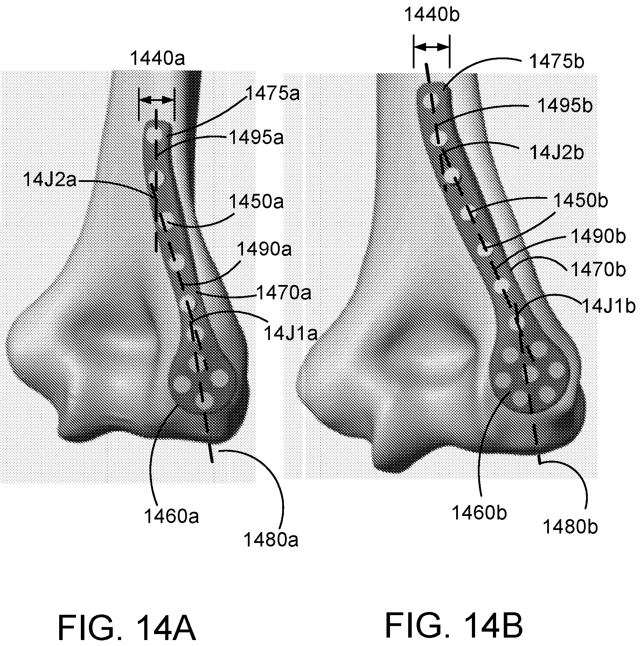

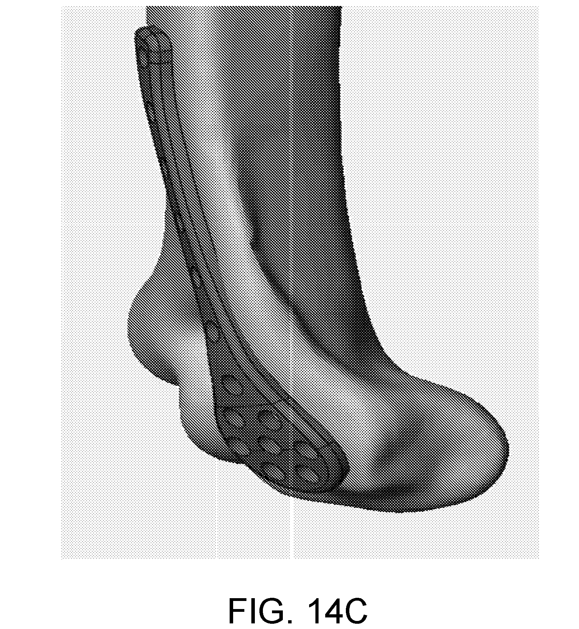

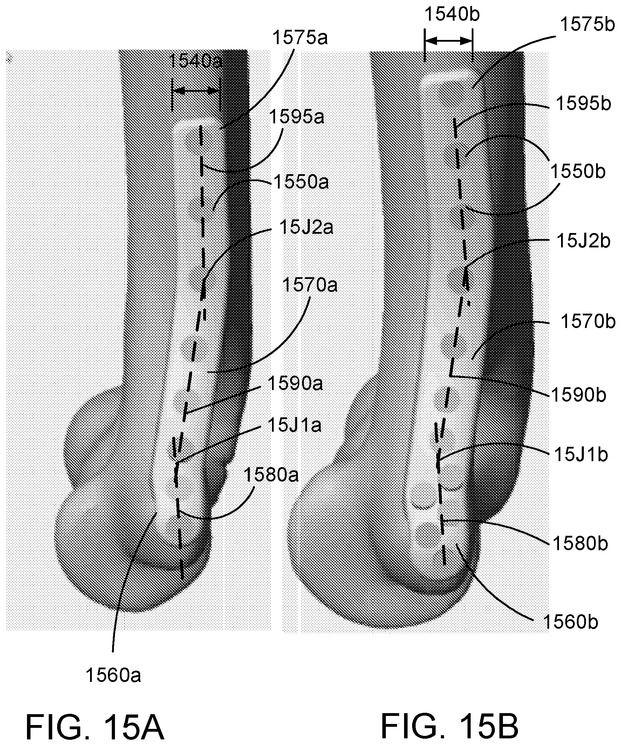

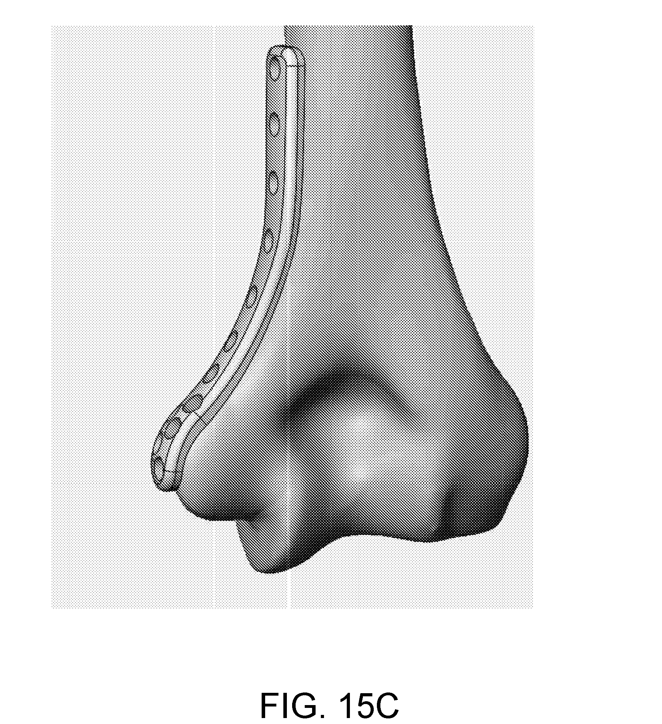

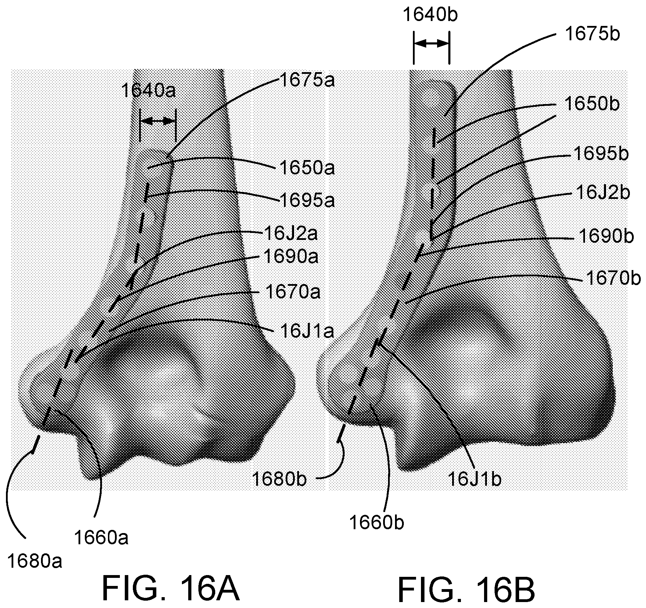

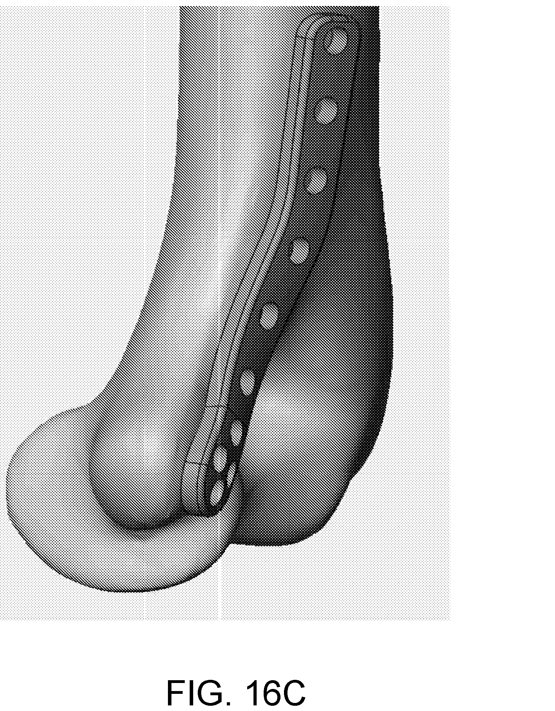

Referring to FIGs. 13A-16C. in one configuration the methodology facilitates developing two different size plate models for each of the four anatomic locations in the distal humerus: lateral (Figs. 13A, 13B, and 13C), posterolateral (Figs. 14A, 14B, and 14C), medial (Figs. 15A, 15B, and 15C), and posteromedial (Figs. 16A, 16B, and 16C). The models were created to test the methodology. In the present example, the methodology resulted in a model that is left and right side specific. One aspect of the models was the ability to maximize distal humeral fixation with a plate that is anatomic in shape and in the appropriate size distribution.

One aspect of fixation of distal humeral fractures is related to the ability to maximize fixation in the distal humeral region, corresponding to distal plate portions 1360a and 1360b, 1460a and 1460b, 1560a and 1560b, and 1660a and 1660b respectively. This is the area where fracture fixation most commonly fails. In addition, this is the region where non-anatomic plates contribute to malposition of the fragments, painful prominence of the plates under the skin, as well as the need for more soft tissue disruption to place the plates. The shape of the models can be extended a variable distance proximally up the humeral shaft with plate extension portion 1370a and 1370b, 1470a and 1470b, 1570a and 1570b, and 1670a and 1670b based on the length of fracture propagation and the length of the plate desired by the company or surgeon.

The shape of the most distal aspect of the plates was designed based on the natural curvature of the bone. This anatomic shape optimizes plate-bone contact, maximizes the ability of the plate to cradle and support the distal humeral region, increases the number of distal screws to improve fixation while at the same time minimizing plate which can cause soft tissue pain and nerve irritation. One can see that each of the two size plates has an optimized number of distal screw holes to maximize fixation in the appropriately sized patient. This is a significant improvement in current designs that have only one size width plate.

The methodology also resulted in defining the arcs of curvature of the distal humeral region improving the ability of the plate to be appropriately placed on the bone minimizing the need for plate bending or importantly, malpositioning the fracture in regard to angulation. In addition, specific angles were developed so that the proximal aspect of the plate sits more in line with the humeral shaft, thereby decreasing the need for soft tissue detachment.

Referring to FIG. 13A and 13B, a lateral distal humeral plate is shown in a sagittal view in two different sizes. Plate extension portion 1370a and 1370b has a longitudinal axis 1390a and 1390b and distal plate portion 1360a and 1360b includes a longitudinal axis 1380a and 1380b. In one embodiment, axis 1390a and axis 1380a intersect at junction 13J1a, and axis 1390b and axis 1380b intersect at junction 13J1b. The angle between axis 1390a and axis 1380a, and the angle between axis 1390b and axis 1380b, may be determined by angle 915 in FIG. 9C. In some embodiments, a second plate extension portion 1375a and 1375b may extend further up the humerus and include a longitudinal axis 1395a and 1395b. Axis 1395a and axis 1390a intersect at junction 13J2a, and axis 1395b and axis 1390b intersect at junction 13J2b. The angle between axes 1390a and 1395a, and 1390b and 1395b, may be determined by angle 914 in FIG. 9C. In some embodiments, the length and width of 1360a and 1360b may be determined by 911 and 913 respectively from FIGs. 9A and 9B; and the length and width of 1370a and 1370b may be determined by 910 and 908 respectively from FIGs. 9A and 9B. Alternatively, angles, lengths, widths, curvature, and other parameters of the plate may be based upon fitting to the anatomy, where a designer may adjust a parameter in order to accommodate a particular patient's or a population's group anatomy. A designer may also take into account both the parameters provided by the method and also engineering (such as material considerations and edge configurations) or anatomic parameters in a design. Referring to FIG. 13C, the lateral plate from FIG. 13A and 13B is shown in a view between the sagittal and coronal planes.

Referring to FIG. 14A and 14B, a posterolateral distal humeral plate is shown in a coronal view in two different sizes. Plate extension portion 1470a and 1470b has a longitudinal axis 1490a and 1490b and distal plate portion 1460a and 1460b includes a longitudinal axis 1480a and 1480b. In one embodiment, axis 1490a and axis 1480a intersect at junction 14J1a, and axis 1490b and axis 1480b intersect at junction 14J1b. The angle between axis 1490a and axis 1480a, and axis 1490b and axis 1480b, may be determined by angle 1025 in FIG. 10B. In some embodiments, a second plate extension portion 1475a and 1475b may extend further up the humerus and include a longitudinal axis 1495a and 1495b. Axis 1490a and axis 1495a intersect at junction 14J2a, and axis 1490b and axis 1495b intersect at junction 14J2b. The angle between axes 1490a and 1495a, and axes 1490b and 1495b, may be determined by angle 1024 in FIG. 10B. In some embodiments, the length and width of 1460a and 1460b may be determined by 1023 and 1020 respectively from FIGs. 10A and 10B; and the length of 1470a and 1470b may be determined by adding together 1021 and 1022 from FIG. 10B; and the width of 1470a and 1470b may be determined by 1019 from FIG. 10A. Alternatively, angles, lengths, widths, curvature, and other parameters of the plate may be based upon fitting to the anatomy, where a designer may adjust a parameter in order to accommodate a particular patient's or a population's group anatomy. A designer may also take into account both the parameters provided by the method and also engineering (such as material considerations and edge configurations) or anatomic parameters in a design. Referring to FIG. 14C, the lateral plate from FIG. 14A and 14B is shown in a view between the sagittal and coronal planes.

Referring to FIG. 15A and 15B, a medial distal humeral plate is shown in a sagittal view in two different sizes. Plate extension portion 1570a and 1570b has a longitudinal axis 1590a and 1590b and distal plate portion 1560a and 1560b includes a longitudinal axis 1580a and 1580b. In one embodiment, axis 1590a and axis 1580a intersect at junction 15J1a, and axis 1590b and axis 1580b intersect at junction 15J1b. The angle between axis 1590a and axis 1580a, and between axis 1590b and axis 1580b, may be determined by angle 1134 in FIG. 11C. In some embodiments, a second plate extension portion 1575a and 1575b may extend further up the humerus and include a longitudinal axis 1595a and 1595b. Axis 1590a and axis 1595a intersect at junction 15J2a, and axis 1590b and axis 1595b intersect at junction 15J2b. The angle between axes 1590a and 1595a, and axes 1590b and 1595b, may be determined by angle 1133 in FIG. 11C. In some embodiments, the length and width of 1560a and 1560b may be determined by 1131 and 1128 respectively from FIGs. 11A and 11B; and the length and width of 1570a and 1570b may be determined by 1130 and 1127 respectively from FIGs. 11A and 11B. Alternatively, angles, lengths, widths, curvature, and other parameters of the plate may be based upon fitting to the anatomy, where a designer may adjust a parameter in order to accommodate a particular patient's or a population's group anatomy. A designer may also take into account both the parameters provided by the method and also engineering (such as material considerations and edge configurations) or anatomic parameters in a design. Referring to FIG. 15C, the lateral plate from FIG. 15A and 15B is shown in a view between the sagittal and coronal planes.

Referring to FIGs. 16A and 16B, a posteromedial distal humeral plate is shown in a coronal view in two different sizes. Plate extension portion 1670a and 1670b has a longitudinal axis 1690a and 1690b and distal plate portion 1660a and 1660b includes a longitudinal axis 1680a and 1680b. In one embodiment, axis 1690a and axis 1680a intersect at junction 16J1a, axis 1690b and axis 1680b intersect at junction 16J1b. The angle between axis 1690a and axis 1680a, and axis 1690b and axis 1680b, may be determined by angle 1244 in FIG. 12B. In some embodiments, a second plate extension portion 1675a and 1675b may extend further up the humerus and include a longitudinal axis 1695a and 1695b. Axis 1690a and axis 1695a intersect at junction 16J2a, and axis 1690b and axis 1695b intersect at junction 16J2b. The angle between axes 1690a and 1695a, and axes 1690b and 1695b, may be determined by angle 1243 in FIG. 12B. In some embodiments, the length and width of 1660a and 1660b may be determined by 1242 and 1239 respectively from FIGs. 12A and 12B; and the length and width of 1670a and 1670b may be determined by 1241 and 1238 respectively from FIGs. 12A and 12B. Alternatively, angles, lengths, widths, curvature, and other parameters of the plate may be based upon fitting to the anatomy, where a designer may adjust a parameter in order to accommodate a particular patient's or a population's group anatomy. A designer may also take into account both the parameters provided by the method and also engineering (such as material considerations and edge configurations) or anatomic parameters in a design. Referring to FIG. 16C, the lateral plate from FIG. 16A and 16B is shown in a view between the sagittal and coronal planes.

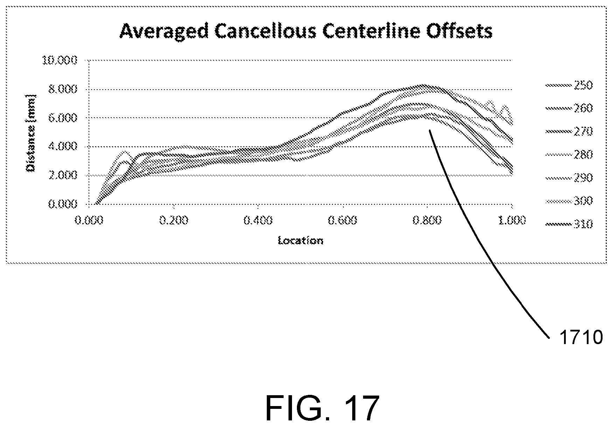

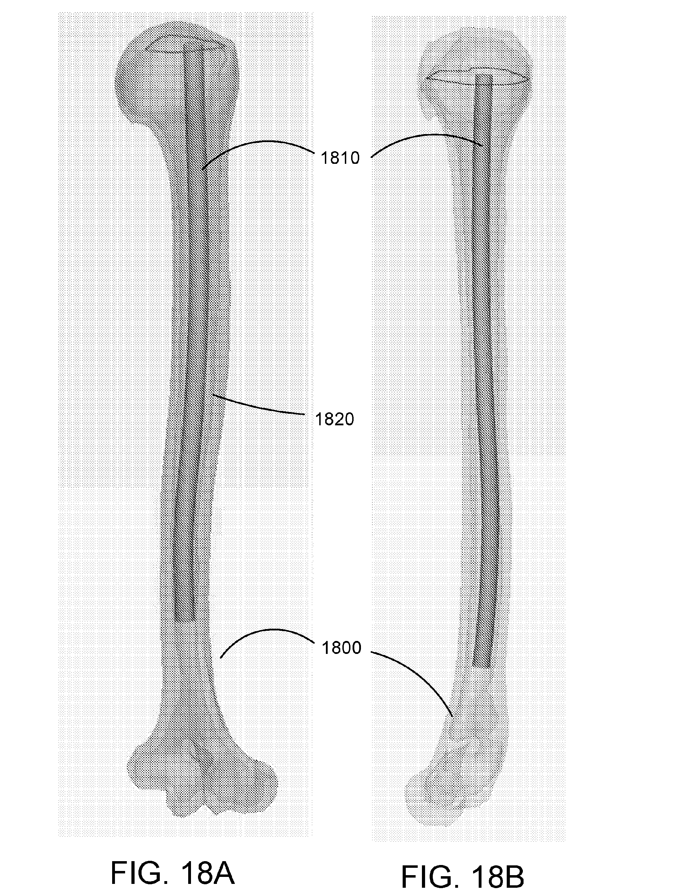

Referring to Figs. 18A and 18B, in one embodiment the analysis of the measurements derived from the methodology resulted in developing an idealized shape of a humeral nail. The humeral nail 1810 includes at least one bent portion 1820 in order to conform to the bends in the bone 1800. The methodology of comparing a cancellous centerline and a straight centerline was used in the design, such that the deviation from the straight longitudinal bone axis 254 (Fig. 2B) from the centerline following longitudinal bone axis 254 (Fig. 2C) may be determined in order to indicated where over the length of the bone the area or areas of greatest bending or deflection take place. A graphical representation of the averaged offsets is shown in Fig. 17, where maximum deviation from the centerline 1710 may provide design considerations for a developer to locate a bend in a nail at that location. This may be used when designing plates, intramedullary nails, stems, or other implants for bends that may be needed to conform to the anatomy. Determining the specific coordinates of the centerlines allows one to know the offset of the two centerlines in direction and magnitude. A summary of the bend locations for different size patients is shown in Table 9.

**Table 9**

| **Increment Bucket [mm]** | **Location of Bend in Bone as [%] of Length** | **Distance [mm]** |
|---|---|---|
| 250 | 76.00 | 6.144 |
| 260 | 81.10 | 6.278 |
| 270 | 79.60 | 6.933 |
| 280 | 82.10 | 6.707 |
| 290 | 81.0 | 7.848 |
| 300 | 80.00 | 8.103 |
| 310 | 79.00 | 8.270 |

In one embodiment, three different length nails were created to highlight the potential of the methodology to optimize design. The models were created to test the methodology. The methodology resulted in an intramedullary nail model that is left and right side specific.

The key aspect of placement of an intra-medullary nail is related to the ability to maximize contact with the inner bone to maximize stability and place the nail without causing a more distal fracture. These are areas where non-anatomic intramedullary nails most commonly fail. The shape of the intramedullary nail models can be normalized to length of the nail desired by the company or surgeon.

### Using Correlation for Expediting Design or Patient Fitment

In some embodiments, the correlation between different measurements allows for rapidly designing implants using the above methods, or allows a surgeon to quickly determine which implant will fit a particular patient using as little as one simple measurement. In one embodiment, a significant relationship exists between the distance from the greater tuberosity to the middle of the deltoid insertion and the overall length of the humerus. This is reflected in Table 10 and allows for a surgeon to measure either the distance from the greater tuberosity to the middle of the deltoid insertion or the overall length of the humerus to determine a plate or nail that will fit a patient, or allows for creating designs based upon one of these parameters using the relationship in Table 10.

**Table 10**

| | **Relationship** | **R-Squared** | **P-value** | **Correlatio n** |
|---|---|---|---|---|
| **Middle DI** | y=107.3+.0784x | 18.59% | 0.002 | 0.43 |
| **Full Length** | y=257.0+1.77x | 26.7% | 0.001 | 0.52 |

In one embodiment, a regression analysis establishes a correlation for posterolateral condyle PLC width 1 vs PLC Maximum Width, with a p-value of 0.001 showing that the relationship is statistically significant. Also, a positive correlation factor of 0.71 indicates that when PLC Maximum Width increases, PLC Width 1 also tends to increase. A fitted linear model that describes this relationship is y=2.845+.5265x.

In one embodiment, a multiple regression model is used to establish a statistically significant relationship (p-value < 0.001) between the full length of the humeral bone and lateral condyle (LC) Maximum Width, the distance from the greater tuberosity to the middle of the deltoid insertion, and gender with an R-squared of 72.95%. The relationship equation to determine full humeral length for female and male are y= 173.1 + 2.098X2 + 0.832X4 and y=186.7 + 2.098X2 + 0.832X4 where X2 is LC Maximum Width and X4 is the greater tuberosity to the middle of the deltoid insertion distance, respectively.

### External Humeral Anatomy: Proximal Humeral Plates and Distal Humeral Plates

The methodology has provided specific insight into both the proximal and distal external humeral anatomy. The methodology revealed a specific pattern and shape of the humerus. The specific lengths, widths, angles, and arcs provide a detailed understanding of the anatomy and can facilitate a truly anatomic plate design. Moreover, the data from this methodology defines a specific range of anatomic sizes to accommodate patients. Rather than forcing the anatomy to adapt to a plate with one width and a non-anatomic shape, the models used to test the methodology confirm an improved fit.

### Internal Humeral Anatomy: Intramedullary Nail and Long Stem Humeral Component

The methodology has also provided unique insight into the complex three dimensional intramedullary anatomy of the humerus. One can see that connecting the center of the proximal and distal humerus in a straight line does not follow a path in the center of the medullary bone down the length of the humerus. Therefore, this explains the challenges of trying to force a straight humeral intramedullary nail or long stem in the humeral canal and the risk of further fracturing. The specific offset from the two centerlines can facilitate the design of a truly anatomic intramedullary design. The data from this methodology defines a specific range of anatomic sizes to accommodate patients.

### Quality Control Applications

In one configuration, the statistical correlations that have been disclosed, including ratios and other relationships between measurements, could be used as an internal quality control for establishing a ground truth that segmentation has been done correctly. Laboratories must perform routine quality control tests, usually every day, and in many cases, several times a day. Quality control tests usually include normal and abnormal samples to ensure that the equipment, the technologist, and the reagents used in the test are performing to established standards. The laboratory must get the right result in order to be allowed to continue to test patient samples. If the lab repeatedly fails to get the right result, it is prohibited from continuing the performance of that test until it can demonstrate that it has corrected the problems that led to the unacceptable results.

For automated segmentation routines, the methodology can be used to keep the machine honest. When an automated routine segments a bone from the image, the methodology may establish what length or width the bone should be in that situation, which the automated routine can take into account and adjust its segmentation as needed. In one non-limiting example, an automated segmentation routine may segment based upon the intensity values of pixels or voxels in an image, but the threshold intensity value used to establish which pixels or voxels are identified as bone may be adjusted based upon input from the methodology presented here. More specifically, adjusting the threshold lower in order to allow more pixels or voxels to qualify as a particular bone or tissue type may be done in order to lengthen or widen a bone in the image by allowing more pixels or voxels at the edge of the bone to qualify as bone material, whereas increasing the threshold value may decrease the length or width of the bone by removing pixels or voxels at the edge of the bone. By increasing or decreasing the number of pixels or voxels that qualify as bone, the length or width of a bone can be matched to a ratio of length to width that the method described above indicates would be appropriate for that anatomy. One skilled in the art will appreciate that instead of lengths and widths, angles, curves, bends, and other bone parameters as discussed above may be used. One skilled in the art will also appreciate that different classification routines may be used instead of intensity thresholds, such as using CT numbers, contrast to noise ratios, signal to noise ratios, texture may analyses and the like. This could be similar to other tests used in medicine where correlations are used to ensure that the testing and/or sample was appropriate.

Thus, the disclosure provides methods to improve understanding of humeral anatomy to facilitate the design and selection of anatomically correct implants and/or periprosthetic bone plates where understanding the periprosthetic fracture may be taken into account. Use of this method and the data that it provides gives unique insight into the number, size and shape of implants or periprosthetic bone plates for shoulder arthroplasty and elbow arthroplasty. This method also provides valuable information for the optimal design, shape, and size of implants or periprosthetic bone plates to maximize healing. In the course of new product development, this method is a valuable resource that can be used to radiographically evaluate each new component design to ensure optimal fit prior to component production and product launch. While the disclosure is described herein as a method for the optimization for humeral shoulder and elbow component design, it can be used for other joints (e.g., hip, knee, elbow, foot, ankle, etc...), such that the same methodology that was developed to understand the external and internal anatomy of the humerus can be similarly applied to other bones, including but not limited to the femur, tibia, fibula, radius, ulna, and vertebral bodies. This methodology would substantially improve the ability to design truly anatomic plates, intramedullary nails, and long stem components for arthroplasty in the appropriate size distribution.

## Claims

1. An implant (510a, 510b, and 510c) for treating a fracture in a bone of a subject, the implant comprising:
a first section (570a, 570b, and 570c) having a first longitudinal axis (590a, 590b, and 590c);
a second section (560a, 560b, and 570c) having a second longitudinal axis (580a, 580b, and 580c);
the first section being connected to the second section thereby defining a first junction (5Ja, 5Jb, and 5Jc) between the first section and the second section, wherein the first junction corresponds to a transition from a first anatomical feature of the bone with the first section, to a second anatomical feature of the bone with the second section, wherein the first anatomical feature is a centerline of the bone and the second anatomical feature is at least one of a tuberosity or a condyle;
the first longitudinal axis and the second longitudinal axis forming an oblique angle at the first junction; and
wherein the second section has a terminal end section having a width greater than the first section and wherein the first section has a length that is greater than a length of the second section;
wherein the first section and the second section are configured to provide fixation to a region of the bone and providing a plurality of screw holes (550a, 550b, and 550c), and
wherein the bone is a humerus that includes a greater tuberosity, and the sloped portion is dimensioned to conform in a lateral view of the humerus to the greater tuberosity of the humerus
**characterized in that** the terminal end section has a flat sloped portion extending from a first curved section at a first end of the width of the terminal end section to a second curved section at an opposite second end of the width of the terminal end section.

2. The implant of claim 1 wherein:
the first junction forms a transition portion dimensioned to provide a curvature (530a, 530b, and 530c) connecting the first section to the second section.

3. The implant of either claim 1 or claim 2, wherein the implant has a bone interface surface that faces the bone when the implant is placed on the bone, and wherein an edge formed by the bone interface surface and the sloped portion is smooth.

4. The implant of either claim 1 or claim 2, wherein the oblique angle is greater than or including 145.5 degrees and less than and including 161.1 degrees.

5. The implant of claim 1 wherein the implant is at least one of a periprosthetic bone plate, a proximal humeral plate, or a distal humeral plate.

6. The implant of claim 1 wherein:
the implant has a bone interface surface (122) that faces the bone when the implant is placed on the bone across the fracture, the bone interface surface having a proximal region proximal to a first plane (P4C) transverse to the implant, a distal region distal to a second plane (P4D) transverse to the implant including a length that is greater than a length of the proximal region, and a midshaft region positioned between the first plane (P4C) and the second plane (P4D) including a junction corresponding to a transition from a first anatomical feature of the bone with the distal region, to a second anatomical feature of the bone with the proximal region, the bone interface surface having a shape that transitions from a first curvature that is convex or concave at a proximal portion (124) of the implant to a second curvature at a second portion of the implant longitudinally adjacent to the proximal portion of the implant, the second curvature being convex when the first curvature is concave, and the second curvature being concave when the first curvature is convex.

## Patentansprüche

1. Implantat (510a, 510b und 510c) zur Behandlung einer Fraktur in einem Knochen eines Patienten, wobei das Implantat umfasst:
einen ersten Abschnitt (570a, 570b und 570c) mit einer ersten Längsachse (590a, 590b und 590c);
einen zweiten Abschnitt (560a, 560b und 570c) mit einer zweiten Längsachse (580a, 580b und 580c);
wobei der erste Abschnitt mit dem zweiten Abschnitt verbunden ist, wodurch eine erste Verbindungsstelle (5Ja, 5Jb und 5Jc) zwischen dem ersten Abschnitt und dem zweiten Abschnitt definiert wird, wobei die erste Verbindungsstelle einem Übergang von einem ersten anatomischen Merkmal des Knochens mit dem ersten Abschnitt zu einem zweiten anatomischen Merkmal des Knochens mit dem zweiten Abschnitt entspricht, wobei das erste anatomische Merkmal eine Mittellinie des Knochens ist und das zweite anatomische Merkmal mindestens eine Tuberositas und/oder ein Kondylus ist;
wobei die erste Längsachse und die zweite Längsachse an der ersten Verbindungsstelle einen schrägen Winkel bilden; und
wobei der zweite Abschnitt einen Endbereich aufweist, der eine größere Breite als der erste Abschnitts hat, und wobei der erste Abschnitt eine Länge aufweist, die größer ist als eine Länge des zweiten Abschnitts;
wobei der erste Abschnitt und der zweite Abschnitt dazu eingerichtet sind, eine Fixierung an einem Bereich des Knochens bereitzustellen und mehrere Schraubenlöcher (550a, 550b und 550c) bereitzustellen, und
wobei der Knochen ein Humerus ist, der eine größere Tuberositas umfasst, und der abgeschrägte Abschnitt so bemessen ist, dass er in einer Seitenansicht des Humerus an die größere Tuberositas des Humerus angepasst ist,
**dadurch gekennzeichnet, dass** der Endbereich einen flachen abgeschrägten Abschnitt aufweist, der sich von einem ersten gekrümmten Abschnitt an einem ersten Ende der Breite des Endbereichs zu einem zweiten gekrümmten Abschnitt an einem gegenüberliegenden zweiten Ende der Breite des Endbereichs erstreckt.

2. Das Implantat nach Anspruch 1, wobei:
die erste Verbindungsstelle einen Übergangsabschnitt bildet, der so bemessen ist, dass er eine Krümmung (530a, 530b und 530c) bereitstellt, die den ersten Abschnitt mit dem zweiten Abschnitt verbindet.

3. Das Implantat nach einem der Ansprüche 1 oder 2, wobei das Implantat eine Knochenkontaktfläche aufweist, die dem Knochen zugewandt ist, wenn das Implantat auf dem Knochen angeordnet ist, und wobei eine durch die Knochenkontaktfläche und den abgeschrägten Abschnitt gebildete Kante glatt ist.

4. Das Implantat nach einem der Ansprüche 1 oder 2, wobei der schräge Winkel größer oder gleich 145,5 Grad und kleiner oder gleich 161,1 Grad ist.

5. Das Implantat nach Anspruch 1, wobei das Implantat mindestens eines aus einer periprothetischen Knochenplatte, einer proximalen Humerusplatte oder einer distalen Humerusplatte ist.

6. Das Implantat nach Anspruch 1, wobei
das Implantat eine Knochenkontaktfläche (122) aufweist, die dem Knochen zugewandt ist, wenn das Implantat über der Fraktur auf dem Knochen angeordnet wird, wobei die Knochenkontaktfläche einen proximalen Bereich proximal zu einer ersten zum Implantat transversalen Ebene (P4C), einen distalen Bereich distal zu einer zweiten zum Implantat transversalen Ebene (P4D) umfasst, wobei der distale Bereich eine Länge aufweist, die größer ist als eine Länge des proximalen Bereichs, und einen mittleren Schaftbereich aufweist, der zwischen der ersten Ebene (P4C) und der zweiten Ebene (P4D) angeordnet ist und eine Verbindungsstelle umfasst, die einem Übergang von einem ersten anatomischen Merkmal des Knochens mit dem distalen Bereich zu einem zweiten anatomischen Merkmal des Knochens mit dem proximalen Bereich entspricht, wobei die Knochenkontaktfläche eine Form aufweist, die von einer ersten Krümmung, die an einem proximalen Abschnitt (124) des Implantats konvex oder konkav ist, zu einer zweiten Krümmung an einem zweiten Abschnitt des Implantats, der in Längsrichtung an den proximalen Abschnitt des Implantats angrenzt, übergeht, wobei die zweite Krümmung konvex ist, wenn die erste Krümmung konkav ist, und die zweite Krümmung konkav ist, wenn die erste Krümmung konvex ist.

## Revendications

1. Implant (510a, 510b, et 510c) pour traiter une fracture dans un os d'un sujet, l'implant comprenant :
une première section (570a, 570b, et 570c) ayant un premier axe longitudinal (590a, 590b, et 590c) ;
une seconde section (560a, 560b, et 570c) ayant un second axe longitudinal (580a, 580b, et 580c) ;
la première section étant reliée à la seconde section définissant ainsi une première jonction (5Ja, 5Jb, et 5Jc) entre la première section et la seconde section, dans lequel la première jonction correspond à une transition d'une première caractéristique anatomique de l'os avec la première section, à une seconde caractéristique anatomique de l'os avec la seconde section, dans lequel la première caractéristique anatomique est une ligne centrale de l'os et la seconde caractéristique anatomique est au moins l'un parmi une tubérosité ou un condyle ;
le premier axe longitudinal et le second axe longitudinal formant un angle oblique au niveau de la première jonction ; et
dans lequel la seconde section a une section d'extrémité terminale ayant une largeur supérieure à celle de la première section et dans lequel la première section a une longueur qui est supérieure à une longueur de la seconde section ;
dans lequel la première section et la seconde section sont configurées pour fournir une fixation à une région de l'os et fournissant une pluralité de trous de vis (550a, 550b, et 550c), et
dans lequel l'os est un humérus comportant une tubérosité supérieure, et la partie inclinée est dimensionnée pour se conformer, sur une vue latérale de l'humérus, à la tubérosité supérieure de l'humérus
**caractérisé en ce que** la section d'extrémité terminale présente une partie plate inclinée s'étendant d'une première section incurvée à une première extrémité de la largeur de la section d'extrémité terminale à une seconde section incurvée à une seconde extrémité opposée de la largeur de la section d'extrémité terminale.

2. Implant selon la revendication 1, dans lequel :
la première jonction forme une partie de transition dimensionnée pour fournir une courbure (530a, 530b, et 530c) reliant la première section à la seconde section.

3. Implant selon la revendication 1 ou la revendication 2, dans lequel l'implant présente une surface d'interface osseuse qui fait face à l'os lorsque l'implant est placé sur l'os, et dans lequel un bord formé par la surface d'interface osseuse et la partie inclinée est lisse.

4. Implant selon la revendication 1 ou la revendication 2, dans lequel l'angle oblique est supérieur ou égal à 145,5 degrés, inclus, et inférieur ou égal à 161,1 degrés, inclus.

5. Implant selon la revendication 1, dans lequel l'implant est au moins l'une parmi une plaque osseuse périprothétique, une plaque humérale proximale ou une plaque humérale distale.

6. Implant selon la revendication 1, dans lequel :
l'implant présente une surface d'interface osseuse (122) qui fait face à l'os lorsque l'implant est placé sur l'os à travers la fracture, la surface d'interface osseuse présentant une région proximale qui est proximale par rapport à un premier plan (P4C) transversal à l'implant, une région distale qui est distale par rapport à un second plan (P4D) transversal à l'implant comportant une longueur qui est supérieure à une longueur de la région proximale, et une région médiane positionnée entre le premier plan (P4C) et le second plan (P4D), comportant une jonction correspondant à une transition d'une première caractéristique anatomique de l'os avec la région distale, à une seconde caractéristique anatomique de l'os avec la région proximale, la surface d'interface osseuse présentant une forme qui passe d'une première courbure, qui est convexe ou concave, au niveau d'une partie proximale (124) de l'implant, à une seconde courbure au niveau d'une seconde partie de l'implant, longitudinalement adjacente à la partie proximale de l'implant, la seconde courbure étant convexe lorsque la première courbure est concave, et la seconde courbure étant concave lorsque la première courbure est convexe.
